# EUROPEAN PATENT SPECIFICATION

(11) **EP 3 258 895 B1**
(45) Date of publication and mention of the grant of the patent: **16.11.2022**
(21) Application number: 16752043.6
(22) Date of filing: 18.02.2016
(51) Int. Cl.: A61M 35/00, A61M 37/00

(54) **REGULATOR DEVICE FOR DRUG PATCH**
REGLERVORRICHTUNG FÜR WIRKSTOFFPFLASTER
DISPOSITIF DE RÉGULATEUR POUR PATCH MÉDICAMENTÉ

(30) Priority: 18.02.2015 US 201514624721
(43) Date of publication of application: 27.12.2017
(73) Proprietor: Alma Therapeutics Ltd., 4951038 Petach-Tilkva (IL)
(72) Inventor: GROSS, Yossi, 7316000 Moshav Mazor (IL); CABIRI, Oz, 4526611 Hod-HaSharon (IL); HEZKIAHU, Ran, 4668346 Herzlia (IL)
(74) Representative: Jaeger, Michael David
(86) International application number: PCT/IL2016/050196
(87) International publication number: WO 2016/132368

(56) References cited:
- WO-A1-2014/203910
- US-A- 5 498 235
- US-A1- 2003 065 294
- US-A1- 2008 208 146
- US-A1- 2013 144 261
- US-B1- 6 589 202

## Description

### FIELD AND BACKGROUND OF THE INVENTION

The present invention relates to a regulator for a transdermal drug administration to a surface of a skin of a subject.

U.S. Patent Publication No. 2014/0323423 discloses "systems and methods for longevity, anti-aging, fatigue management, obesity, weight loss, weight management, delivery of nutraceuticals, and treating hyperglycemia, Alzheimer's disease, sleep disorders, Parkinson's disease, Attention Deficit Disorder and nicotine addiction involve synchronizing and tailoring the administration of nutraceuticals, medications and other substances (for example, stimulants) in accordance with the body's natural circadian rhythms, meal times and other factors. Improved control of blood glucose levels, extended alertness, and weight control, and counteracting of disease symptoms when they are at their worst are possible. An automated, pre-programmable transdermal administration system is used to provide pulsed doses of medications, pharmaceuticals, hormones, neuropeptides, anorexigens, pro-drugs, stimulants, plant extracts, botanicals, nutraceuticals, cosmeceuticals, phytochemicals, phytonutrients, enzymes, antioxidants, essential oils, fatty acids, minerals, vitamins, amino acids, coenzymes, or other physiological active ingredient or precursor. The system can utilize a pump, pressurized reservoir, a system for removing depleted carrier solution, or other modulated dispensing actuator, in conjunction with porous membranes or micro-fabricated structures."

U.S. Patent Publication No. 2014/0207048 discloses "establishing a bio-synchronous treatment protocol that incorporates individual temporal and innate biological characteristics into a pharmacological treatment plan. The bio-synchronous treatment protocol is thereafter initiated using bioactive agent delivery device. Bio-synchronous drug delivery includes continual collection of patient data such as physical, psychological, temporal and environmental characteristics. This data is analyzed so to not only determine an initial treatment protocol but to also determining whether modification to the ongoing bio-synchronous treatment protocol is required. And, responsive to determining a modification is required the system modifies the bio-synchronous treatment protocol and use of delivery device. These modifications and treatment protocols can include reactive and proactive psychological support supplied to the patient in a variety of formats."

U.S. Patent Publication No. 2014/0207047 discloses "a bioactive agent delivery device having a plurality of reservoirs wherein each reservoir houses a solvent provides a means for separable and segregated delivery of bioactive agent(s) to a patient. The device can include a plurality of absorbent materials wherein each absorbent material is pretreated with a bioactive agent and a delivery mechanism operable to deliver to any of the plurality of absorbent materials a controlled portion of solvent from any of a plurality of reservoirs. A diffusion layer interposed between the absorbent materials and an epidermis transfers the bioactive agent from the absorbent materials to the epidermis for delivery of the bioactive agent."

U.S. Patent Publication No. 2014/0200525 discloses "systems and methods for synchronizing the administration of compounds with the human body's natural circadian rhythms and addiction rhythms to counteract symptoms when they are likely to be at their worst by using an automated and pre programmable transdermal or other drug administration system."

Additional background art includes U.S. Patent Publication No. 2012/0302942 U.S. Patent Publication No. 2008/0220092.

US 6,589,202 describes a device for delivering and withdrawing a substance from a patient which includes a support member, a skin penetrating device and an advancing assembly for advancing the skin penetrating device to an operating position. The advancing assembly includes a supply spool for supporting a web having the skin penetrating devices attached thereon and a take up spool for receiving the web with the spent skin penetrating devices.

WO 2014/203910 describes an applicator for applying microneedles having a rolling system for bending the microneedles and meking them penetrate the skin.

### SUMMARY OF THE INVENTION

Unless otherwise defined, all technical and/or scientific terms used herein have the same meaning as commonly understood by one of ordinary skill in the art to which the invention pertains.

In addition, the materials, methods, and examples are illustrative only and are not intended to be necessarily limiting.

According to the invention, there is provided a regulator for a transdermal drug administration to a surface of a skin of a subject as defined in claim 1.

### BRIEF DESCRIPTION OF THE SEVERAL VIEWS OF THE DRAWING(S)

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:
FIG. 1 is a flow chart illustrating a method of delayed engagement of an active surface of a medicine patch in accordance with some embodiments of the present invention;
FIG. 2 is a flow chart illustrating a method of disengaging an active surface of a medicine patch in accordance with some embodiments of the present invention;
FIG. 3 is a flow chart illustrating a method of reversibly engaging and disengaging an active surface of a medicine patch in accordance with some embodiments of the present invention;
FIG. 4 is a block diagram illustrating a device for controlling engagement of a drug patch in accordance with an embodiment of the current invention;
FIGs. 5 A, 5B are block diagrams illustrating further optional features of a device for controlling engagement of a drug patch in accordance with some embodiments of the current invention;
FIG. 6 is a block diagram illustrating further optional features of a device for controlling engagement of a drug patch in accordance with an embodiment of the current invention;
FIG. 7 is a flow chart illustration of a method of loading a patch to a dosage control device in accordance with an embodiment of the present invention
FIGs. 8A-8C are perspective side views of engaging and/or disengaging portions of a patch and/or storing disengaged portions of the patch in accordance with arrangements of the disclosure;
FIG. 9A is a perspective view of a ventral side of a patch control device with a roller in accordance with an embodiment of the present invention;
FIG. 9B is a perspective view of a dorsal side of a patch control system with a roller in accordance with an embodiment of the present invention;
FIG. 10 is a photograph of an exemplary patch control device 1001 in use in accordance with an embodiment of the current invention;
FIG. 11A is a block diagram illustration of a patch in accordance with an arrangement of the disclosure;
FIG. 11B is a block diagram illustration of a two part patch in accordance with arrangements of the disclosure;
FIG. 12 is a perspective illustration of a dorsal side of a patch in accordance with arrangements of the disclosure;
FIG. 13 is a perspective illustration of a ventral side of a patch in accordance with arrangements of the disclosure;
FIG. 14 is a schematic illustration of an adapter for connecting a patch to a patch control device in accordance with arrangements of the disclosure;
FIGs. 15A-15C are a perspective views a roller patch control device in accordance with an embodiment of the present invention;
FIGs. 16A-16E illustrate a transdermal delivery assembly, having a curved continuous belt substrate in accordance with arrangements of the disclosure;
FIG. 17 illustrates an optional set of drive interface elements in accordance with arrangements of the disclosure;
FIGs. 18A-18C illustrate progressive movement of a continuous belt substrate in accordance with an arrangement of the disclosure;
FIG. 19 illustrates a patch engagement device according to an arrangement of the disclosure;
FIGs. 20A-20B illustrate a transdermal delivery belt assembly constructed and operative in accordance an arrangement of the disclosure;
FIGs. 21A-21D illustrate a transdermal control device in accordance with an arrangement of the disclosure;
FIGs. 22A-22B illustrate a patch control device with a ductile substrate in accordance with an arrangement of the disclosure;
FIGs. 23A-23B illustrate a patch control device in accordance with an arrangement of the disclosure;
FIG. 24 illustrates a ductile substrate in accordance with an arrangement of the disclosure; and
FIGs. 25A-25B illustrate an expandable substrate in accordance with an arrangement of the disclosure.

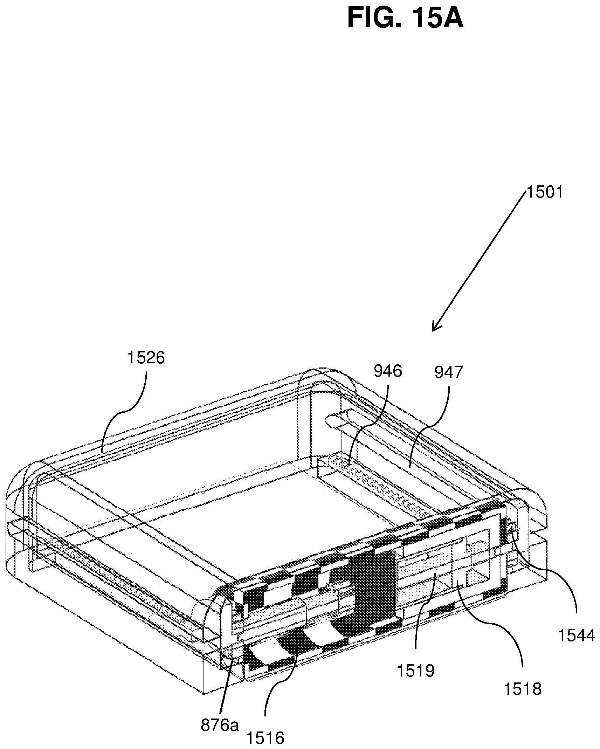

The invention is described in Figs.15A-15C, other embodiments mentioned herein do not form part of the the present invention and are left for illustrative purposes. The methods herein do not form part of the invention and are left for illustrative purposes.

### DESCRIPTION OF SPECIFIC EMBODIMENTS OF THE INVENTION

The present invention relates to a regulator for a transdermal drug administration to a surface of a skin of a subject.

### OVERVIEW

An aspect of some embodiments of the current invention relates to an assembly for controlling timing and/or rate of transdermal delivery that optionally controls drug delivery from a conventional transdermal drug patch. Optionally the system may be used to automatically stop and/or slow delivery of a drug from a transdermal patch.

In some embodiments the device may control delivery from a convention patch approved by a regulatory agency (for example the U.S. Food and Drug Administration FDA and/or the European Commission CE) for a fixed and/or predetermined delivery rate schedule and/or dosage of a drug. For example, in some embodiments, a method and/or apparatus according to the current invention may facilitate use of the patch for delivery of a dose less than the approved dosage and/or at a delivery rate slower than the approved rate.

In some embodiments, delivery may be interrupted before the approved dose has been delivered. Optionally, interrupting delivery may include discontinuing delivery temporarily, discontinuing delivery permanently, and/or reducing a rate of delivery with respect a predetermined and/or fixed rate schedule of a patch. For example, delivery may be automatically interrupted after less than 1% of the approved dose has been administered and/or after delivery of between 1 to 10% and/or between 10 to 30% and/or between 30 to 50% and/or between 50 to 70% and/or between 70 to 90% of the approved dosage. For example, interrupting delivery may include stopping delivery and/or reducing delivery to a rate less than 1% of an approved rate at a given time after application and/or less than 10% and/or between 10 to 30% and/or between 30 to 50% and/or between 50 to 70% and/or between 70 to 90%.

In some embodiments, interruption of delivery is made automatically, for example, without human supervision and/or intercession after applying of the drug patch. Optionally, delivery is made automatically according to preprogramming prior to and/or during use of the device Optionally, a conventional and/or fixed dose and/or regulatory approved patch may be used to administer a drug without any material intervening between the skin and an active surface of the patch and/or without modifying the drug delivery mechanism of the patch. For example, the conventional and/or fixed dose and/or regulatory approved patch may be used to administer a drug without requiring any regulatorily significant substance intervening between the patch and the skin and/or without compromising the approved drug delivery mechanism of the patch.

In some embodiments a dosage schedule and/or a schedule of patch engagement and/or disengagement, and/or control logic (e.g. in reaction to sensors) may be programmed into a device by a supplier of the device and/or a supplier of the drug, for example before distribution. Alternatively a device may be programmed by a medical professional and/or a personal aid and/or a pharmacist and/or the subject before use. Alternatively a device may be programmed by a medical professional and/or a personal aid and/or a pharmacist and/or the subject dynamically and/or during use.

An aspect of some embodiments of the current invention relates to initiating and/or increasing a rate of administering of a transdermal drug automatically. For example, administration may be initiated more than 1/2 hour and/or between 1/2 hour and 2 hours and/or between 2 hours and 8 hours and/or between 8 hours and 24 hours after human intercession or supervision (for example after the system has been placed on a subject). For example, administration may be initiated in response to an event and/or at a fixed time. For example, administration may be initiated while a subject is asleep and/or incapacitated. For example administration may be initiated without supervision and/or without the presence of a helper and/or medical aid.

In some embodiments transdermal drug administration may be automatically initiated from a conventional and/or fixed dosage and/or fixed rate schedule patch. For example, after automated initiation of delivery, transdermal delivery may occur from the patch in accordance to convention regulatory conditions. For example, there may be no regulatorily significant modification to the drug administering portion of the patch. For example, there may be no regulatorily significant intervening substance between the patch and the skin of the subject. Optionally initiation of drug delivery may include switching from no significant delivery to delivery of a significant quantity of the drug. For example, a significant quantity of a drug may include administration at a regulatorily approved rate from a convention drug patch and/or between 50 to 100% of the approved rate and/or between 10 to 50% of the approved rate. In some embodiments initiating delivery may include a significant increase of a rate of delivery. Increasing a rate of delivery may include increasing a rate to between 120% to 150% and/or between 150 to 200% and/or between 200 to 400% more than 400% the rate previous to the increase. For example the rate may be defined as the rate averaged over 1 minute and/or over 5 minutes and/or over 1/2 hour.

In some embodiments, transdermal drug administration may be initiated and/or increased in rate and/or interrupted and/or reduced in rate in response to an event. For example, a sensor may sense an event and trigger a change in the administration. In some embodiments, the change may take place automatically. For example, the sensor may sense a medical condition, for example a change in cardiac function and/or breathing and/or temperature and/or a reaction to a drug. A patch control device may change the administration in response to output of the sensor. For example, sensor output may be received by a processor that may send a signal to a driver to change the drug administration. For example, a sensor may sense a reaction to a drug and send a signal to a processor which may respond by sending a command to interrupt and/or reduce drug administration. For example, a sensor may sense a medical condition requiring a treatment and send a signal to a processor which may respond by sending a command to initiate and/or increase drug administration. For example, a control device may engage a Nicotine patch to increase the Nicotine level in the blood just before a subject a wakes of. In some embodiments, automatically administering Nicotine before wake up may help reduce morning craving for a cigarette. For example, the wakeup time may be fixed slightly before an alarm time set on an alarm clock (for example a wake up alarm of a smart phone). Alternatively or additionally, wake up may be determined by a sensor sensing body movements and/or vital signs. In some embodiments, applying the patch and/or administering a drug close to the time when it is needed may increase the effect of the drug without increasing the dosage. In some embodiments, administering the drug close to the time of need may facilitate reduction of dosage and/or reduce the habituation effect of high levels of drug administration and/or reduce the risk for over dose.

In some embodiments, a conventional patch may include a passive delivery mechanism. For example, a convention transdermal patch may include a drug-in-adhesive monolithic patch and/or a drug-in-adhesive multilaminate patch and/or a liquid reservoir patch and/or a polymer matrix patch. The patch optionally may be based on a reservoir system and/or a matrix system with and/or without a rate controlling membrane.

An aspect of some embodiments of the current invention relates to automated engaging and/or disengaging of a transdermal drug patch. In some embodiments, an apparatus may automatically engage and/or disengage a drug patch at a fixed time and/or in response to an event. Alternatively or additionally, the apparatus may engage and/or disengage the patch in response to a command.

In some embodiments, the apparatus moves the patch to one or more of a plurality of operational relationships with the skin of the subject (for example a patient). For example, operational relationships may include the patch and/or an active surface of the patch being in full engagement with the skin (i.e., the entire active surface of patch is in contact with the skin), being in partial engagement with the skin (i.e., only a portion of the active surface of patch is in contact with the skin), and being disengaged from the skin (e.g. the active surface of the patch is not in contact with the skin).

In some embodiments, the assembly uses any commercially available transdermal patch impregnated or otherwise provided with a substance. For example, the system can use an existing approved transdermal patch from an approved patch supplier with the previously unknown feature of controlled time of initiation of delivery, breaks in delivery, changes in rate of delivery and/or cessation of delivery.

In some embodiments, the device may disengage a patch by moving an active surface of the patch and/or a portion thereof away from a skin of the subject. For example the active surface may be moved between 0.1 and 1 mm away from the skin and/or between 1 mm and 5 mm and/or between 5 mm and 2 cm and/or more.

In some embodiments, a patch may be peeled from the skin and/or an edge of a patch maybe peeled from the skin. As used herein, the term/phrase peel means to remove and/or lift a covering (e.g. a patch and/or a substrate) from a surface (for example skin) along a line and/or curve (the line or curve will be referred to as a separation line) progressively. As used herein, the term/phrase peel an edge means to remove and/or lift a covering from a surface along a separation line progressively and wherein there is a path from the separation line to an edge of the covering along a separated portion of the covering. As used herein, the term/phrase peel from an edge means to remove and/or lift a covering from a surface along a separation line and wherein there is a path from the separation line to an edge of the covering along a separated portion of the covering and the separation line progresses away from the edge. For example the separation line may progress away from a peeled edge.

In some embodiments, the patch may be engaged by placing on the skin progressively. For example a separation line may serve as a connecting line and may progress from a connected edge toward a separated edge. Optionally, a roller may roll across a patch to separate it or join it to the skin. For example, the separate line may be the line of contact where the patch is sandwiched between the roller and the skin.

In some embodiments a patch may be peeled and/or an edge of the patch may be peeled and/or the patch may be peeled from an edge thereof from the skin. For example, an edge of the patch may be connected to a control device and/or a puller mechanism. Optionally, the active surface of the patch may be lifted progressively off the skin. In some embodiments a patch may be raised off the skin by expanding a variable thickness mesh interceding between the patch and the skin.

In some embodiments a patch may be stored. Optionally the patch may be stored in a disengaged state, for example with an active surface of the patch distanced from the skin of the subject. For example, a patch may be stored rolled onto a roller and/or in a space in a housing and/or covered by a protective liner.

In some embodiments, the system of the invention may engage a patch by applying it to the skin and/or disengage a patch by peeling the patch from the skin. Engaging and/or disengaging may occur at certain times for certain time durations. For example a patch may be engaged and/or disengaged while the user is asleep or incapacitated. In some embodiments, the rate of drug administration may be increased by increasing the active area of the patch in contact with the skin. In some embodiments, the rate of drug administration may be decreased by decreasing the active area of the patch in contact with the skin. The total amount of substance administered is optionally controlled by independently controlling of the time and rate of delivery.

In some embodiments, a patch may have various zones. Disengaging an active surface may include moving the patch such that the active surface is no longer in contact with the skin and/or moving the patch such that a secondary zone contacts the skin. Optionally the secondary zone will contact the same region and/or a different region than the active surface. In some embodiments, an active surface may be partially engaged. For example engaging the active surface and/or increasing the rate of drug delivery may be achieved by increasing the surface area of contact between the active surface and the skin. For example disengaging the active surface and/or decreasing the rate of drug delivery may be achieved by decreasing the surface area of contact between the active surface and the skin.

In some embodiments, a secondary region may be a passive region (e.g. without a drug). Alternatively a secondary region may include a different dosage and/or a different drug and/or different treatment means than the active region. In some embodiments, a patch and/or a substrate may include multiple regions with different drugs and/or treatments. In some embodiments, different zones may contact the skin sequentially and/or in an arbitrary order (for example determined by a command and/or programming of a controller) and/or simultaneously. Optionally a zone may be engaged reversible and/or stored and/or protected during storage and/or reengaged. Optionally reengaging a zone may include reversing a driver and/or a zone may be reengaged by a cyclic system (for example a continuous belt). In some embodiments, a device will progressively engage more active area of a patch, for example to maintain a steady and/or constant dosage.

In some embodiments a conventional patch may be repeatedly engaged and/or disengaged. For example a patch may be engaged and/or disengaged once and/or between 2 to 3 times and/or between 4 to 6 times and/or more. Alternatively or additionally, a patch may be modified for enhanced reapplication. For example, a patch may use a gel instead of and/or in addition to a conventional adhesive to hold the patch in place. In some embodiments gel may enhance reengaging of the patch. For example, a gel patch may be engaged and/or disengaged between 1 to 3 times and/or between 4 to 6 times and/or between 7 to 10 times or more.

In some embodiments, a control device may exert a force relative to a patch and the skin of the subject. For example, an actuator may be connected to a frame. The actuator optionally moves with respect to the frame. Optionally the actuator is connected to a mobile portion of the patch. In some embodiments the frame is attached to the skin of the subject. Optionally, an immobile portion of the patch may attach the frame to the skin. A force between the frame and the actuator optionally results in a force and/or movement of the patch with respect to the skin. Optionally, a driver may exert the force between the frame and the actuator.

In some embodiments a patch control device includes a frame and a roller. In some embodiments, one portion of the patch, for example a leading edge of a mobile section thereof, may be attached to a roller. For example, a stationary portion of the patch may be attached to a frame of the device. For example, a trailing edge of the mobile portion of the patch may be connected to the immobile portion of the patch.

In some embodiments a length of a device may range for example between 5 to 25mm and/or between 25 to 60mm and/or between 60 to 90mm and/or between 90 to 120mm and/or between 120 to 200mm and/or greater than 200mm. In some embodiments a width of a device may range for example between 10 to 50% and/or between 50 to 75% and/or between 75 to 125% and/or between 125 to 200% and/or between 200 to 400% and/or between 400 to 1000% its length. For example, the ventral surface area and/or skin contact surface area of a device may range between 100 to 130% and/or between 130% to 250% and/or between 250% to 500% the area of a ventral surface of a patch to be controlled. For example the device may control a patch of length between 5 to 25mm and/or between 25 to 60mm and/or between 60 to 100mm and/or between 100 to 200mm. In some embodiments a width of a patch may range for example between 10 to 50% and/or between 50 to 75% and/or between 75 to 125% and/or between 125 to 200% and/or between 200 to 400% and/or between 400 to 1000% its length. For example a patch may include a 70x70mm or 50x50mm or 35x35mm patch. Exemplary patches include Nicotrol of GSK and/or Nicoretee and/or Nicoderm. For example a patch may be rated at a fixed dose of about 5, 10, or 15 or 22mg/day over 16 -24 hrs. In some embodiments, a controller will initiate the drug delivery for example between 5 minutes to an hour and/or between an hour and 2 hours and/or between 2 hours and 4 hours and/or between 4 hours and 8 hours and/or between 8 hours and 16 hours and/or between 16 hours and 24 hours after attaching the system to the subject. In some embodiments, the device will move a patch from a disengaged state to an engaged state in a time ranging between 1 seconds to 10 seconds and/or between 10 seconds to 30 seconds and/or between 30 seconds and 90 seconds and/or between 90 seconds and 2 minutes and/or between 2 minutes and 1 hour. Alternatively or additionally, a device may slowly engage a patch over a time ranging between an hour to 4 hours and/or 4 hours to 12 hours and/or 12 hours to 24 hours and/or 24 hours to a week. For example slowly engaging a patch may keep a constant rate of delivery over a period. In some embodiments, drug delivery may start immediately up to a full dose. In some embodiments, the device will move a patch from a engaged state to a disengaged state in a time ranging between 1 seconds to 10 seconds and/or between 10 seconds to 30 seconds and/or between 30 seconds and 90 seconds and/or between 90 seconds and 2 minutes and/or between 2 minutes and 1 hour. For example, disengagement may include pulling a patch from the skin. For example, disengagement may take 10 to 60 seconds if using a motor or 1 to 5 seconds using a preloaded spring and/or gas pressure.

In some embodiments, disengaging a patch (for example, pulling a patch from the skin) and/or storing the patch, may happened at any stage during the delivery. In some embodiments the patch may be stored for reuse within an approved time. In some embodiments, the patch may be replaced and/or the device reused. Alternatively or additionally the patch and device may be replace together. For example a patch and/or a device may be replaced after a time ranging between 1 hour to 8 hours and/or 8 hours to 16 hours and/or 16 hours to 1 day and/or 1 day to 2 days and/or 2 days to a week.

In some embodiments, a portion of the patch may be stored in a disengaged state. For example, between 0 to 20% and/or between 20 to 50% and/or between 50 to 80% and/or between 80 to 100% of the patch may be stored. For example, between 0 to 20% and/or between 20 to 50% and/or between 50 to 80% and/or between 80 to 100% of a mobile portion of patch may be stored.

In some embodiment a roller may engage and/or disengage a patch. Optionally the patch may be stored rolled up on the roller in a disengaged state. For example the roller may unroll the patch on to the skin engaging the patch. Optionally, the roller may roll along the patch, pulling up the patch along a separation line, peeling the patch off the skin and/or disengaging the patch and/or rolling up the patch onto the roller. Optionally, the separation is the line of contact where the patch is sandwiched between the roller and the skin. Optionally, a roller may serve one, some, or all of the functions described above. For example, a roller may serve one, some, or all of guiding movement of a separation line between a patch and skin, pulling a patch away from skin and/or a separation line, and/or storing a patch.

In some embodiments a roller may be configured to rotate at a rate that varies according to the diameter of the roll of patch being rolled up or unrolled. For example, the rate of rotation may be controlled to let out and/or take up the patch at the same rate as the linear movement of the roll. For example, for a larger roll the rate of rotation of the roller may be adjusted downward with respect to the linear rate of progress of the roller. For example, the roller without the patch may have a diameter ranging between 2 to 6 mm and/or between 6 to 10 mm and/or between 10 to 15 mm and/or between 15 to 25 mm. Optionally the diameter of the roll (including the roller with the patch rolled around it) may range between 0 to 2 mm and/or between 2 to 4 mm and/or between 4 to 6 mm and/or between 6 to 10 mm or between 10 to 20 mm greater than the diameter of the roller alone. For example, a patch may be rolled around a roller in 1 layer and/or 2 to 4 layers and/or 4 to 10 layers and/or 10 to 20 layers.

In some embodiments, a patch may be mounted on a substrate and/or an adaptor. The substrate is optionally driven by a driver. Optionally, the operation of the driver is controlled by a controller. For example, the controller may control when and for how long the patch is in a particular operational relationship with the skin (e.g., at what time of the day is the patch in full, partial or no contact with the skin and for what duration of time). Alternatively or additionally, the substrate may be arranged to determine a timing or dosage of the medicine. For example, the driver may drive the substrate at a fixed rate and/or active surfaces may be arranged on the substrate such that they are engaged at predetermined times and/or removed at predetermined times. For example, a driver may include a DC motor and/or a stepper motor and/or a linear actuator and/or a rubber band and/or a spring and/or a compressed gas drive. Alternatively or additionally a patch control device may be manually operated.

In some embodiments, the system may be operated based on a real time clock. Alternatively or additionally the system may be operated remotely, for example with a wireless/cellular connection. For example a parent and/or caretaker may use a cellular connector to remotely control drug administration to children or the elderly. For example, a patch may be stored in the assembly in a disengaged state (no substance administration) and then later engaged (that is, moved to an operational position where the substance is delivered) by authorized personnel (e.g., parent or caregiver).

An aspect of some embodiments of the current invention relates to a method for mounting a transdermal drug patch to a control device and/or a substrate and/or an adapter for mounting to the device. In some embodiments a patch may have a stationary portion and/or a mobile portion.

In some embodiments, the mobile portion of the patch may be engaged and/or disengaged to and from the skin by the patch control device. For example, a mobile portion of the patch may include one or more active and/or inactive surfaces and/or zones. Optionally a stationary portion of a patch may include an inactive zone. Alternatively or additionally a stationary portion of the patch may include one or more active surfaces. Optionally, a stationary portion of the patch may be held continuously engaged to the skin. Alternatively or additionally a stationary portion of the patch may be held continuously distanced away from the skin.

In some embodiments a patch may be attached to a substrate and/or an adaptor. For example, an active surface of the patch may be attached to a mobile portion of the adaptor. Optionally, the mobile portion of the adapter may be engaged and/or disengaged to and from the skin by the patch control device. Optionally the adaptor may include a stationary zone. Optionally, part or all of a stationary portion of the adaptor may be held engaged to the skin. Alternatively or additionally part or all of a stationary portion of the adaptor may be held distanced away from the skin.

In some embodiments, a mobile portion of a patch and/or an adaptor may be separable from a stationary portion thereof. For example, a point on the mobile portion may be distanced from a point on the stationary portion by a given distance along the surface of the substrate. When the portions are separated the distance between the points may be increased over the given distance. For example, when the substrate is lying flat on a surface (for example on a flat surface) a point on the mobile portion may be distanced from a point on the stationary portion by a given minimum distance along the surface. When the portions are separated, the minimum distance between the points may be increased over the given distance along the surface between the points when the portions are not separated. Optionally a discontinuity in the patch may intervene between the mobile region and the stationary region. For example the mobile reason may be separated from the stationary region by the discontinuity. For example, the discontinuity may include a cut and/or a cut out. For example the length of the cut and/or cut out may range between 1 to 5 mm and/or between 5 mm to 2 cm and/or between 2 to 5 cm and/or between 5 to 10 cm greater than 10 cm.

In some embodiments the stationary region may fully or partially surround the mobile region. Optionally the stationary region may not be convex. For example there may be two points located in the stationary region such that a line connecting the points along the surface of the substrate passes through the mobile region. For example there may be two points located in the stationary region such that, when the patch is lying flat on a surface a line connecting the points along the surface passes through the mobile region. For example, the minimum distance between a point in the mobile region and a point in the stationary region when separated may range between 1 mm to 5 mm more than the minimum distance between the points along the surface of the substrate when they are not separated and/or between 5 mm to 2 cm more and/or between 2 cm to 5 cm and/or 5 to 12cm more than the shortest distance between the points along the surface of the substrate when the regions are not separated. For example, the minimum distance between two points when portions of the patch are separated may range between 1 mm to 5 mm more than the minimum distance along a surface onto which the patch is spread when the portions of the patch are not separated and/or between 5 mm to 2 cm more and/or between 2 cm to 5 cm and/or 5 to 12cm more than the shortest distance between the points along the surface.

In some embodiment a substrate may have an adhesive on two opposite faces. For example there may be adhesive on a ventral side of the substrate for connecting to a skin of a subject and/or there may be adhesive on a dorsal side of the substrate for connection to a control device In some embodiments a discontinuity may intercede between a point on the mobile portion and a point on a stationary portion such that when the patch is laid on a surface, the distant along the surface between the points without crossing the discontinuity is greater than the distance along the surface between the two points along the surface crossing the discontinuity by between 1 mm to 5 mm and/or between 5 mm to 10mm and/or between 10 to 50 and/or between 50 to 100 mm. A discontinuity may include for example a cut and/or a score and/or a scratch and/or a fold and/or a slit.

In some embodiments an alignment device is supplied to facilitate alignment of a patch and/or an adaptor and/or a patch control device. Alignment may be adjusted, for example, in the lateral and/or longitudinal position and/or angular alignment. For example, achieving accurate longitudinal position may facilitate dosage control when an active surface is only partially engaged. The exact position of a patch on a device and/or on a substrate and/or on an adapter may be monitored. A patch may be optionally attached to a substrate, for example by adhesive, ultrasonic welding, heat etc. For example an alignment device may guide movement of a patch and/or patch controller device as the patch is being mounted of the device. The alignment device may optionally have a contoured surface that holds a part of a patch extended and/or recessed to match a contact service of a control device.

In some embodiments a device may be sized between 70x70mm to 100x100mm. Optionally the device is adapted for a large dermal patch. For example the device may control a patch having dimensions up to 70x70mm. Alternatively the device may control a smaller patch for example 50x50mm or 35x35mm and/or of various shapes for example circular shape with diameter range 20mm-70mm. Optionally for smaller patches, for example of dimensions between 50x50mmm to 35x35mm and/or smaller (e.g. for lower doses between 3-10mg) a device may be smaller, for example between 70x70mm to 60x60mm and/or smaller.

In some embodiments a control device may use a force to peel a patch from skin. For example a driver may exert a force on an actuator and/or a puller to puller. For example the force may turn a roller and/or move an actuator. For example, the force may range for example between 0.5 and 1 g per mm of peeling line. For example for a 70x70mm patch the total force may range between 350 to 700 g. Alternatively or additionally the force may range between 0.1 and 0.5 g per mm peel line and/or between 1 g/mm to 3 g/mm. The force on the patch is optionally balanced by a counter force on the skin of the subject. For example, the driver may be supported on a frame of the device which is optionally attached to and/or supported by the skin of the subject.

An aspect of some embodiments of the current invention relates to protecting a patch while it is being stored in a control device. For example a liner may be supplied to separate between an active surface of the patch and a storage surface. For example, when the patch is rolled up during storage, the liner may separate between a ventral surface of one layer of the patch and a dorsal surface of the previous layer of the patch. Alternatively or additionally a liner may be part of a device. Optionally, the linear may be made of a non-stick and/or non reactive substance. Optionally, the liner may be flexible and/or impermeable.

In some embodiments, a non-reactive material of a layer, coating, surface and/or liner may be compliant against United States Food and Drug Administration (FDA) Code of Federal Regulations (CFR) 21 (for example subsection 175.300 of section 175 and/or subsection177.1550) and/or European Council (EC) directives and/or regulations for example (EC) 2023/2006 and/or (EC) 1935/2004 and/or (EC) 1272/2008. Optionally the coating is non-oxidizing. Optionally, the material may be dense and/or nonporous for example to prevent growth of mold and/or bacteria. For example the material may produce little or no residual when exposed to water and/or heptanes and/or alcohol (for example 8%) for between 1 to 24 and/or 24 to 150 hours at temperature between -20 to 50 degrees C and/or between 50 to 100 degrees C and/or between 100 to 200 degrees C at normal and/or high pressure. For example residuals may be less than 150parts per million (ppm) and/or less than 50 ppm and/or less than 2 ppm. For example, a non-reactive material may include Polyethylene and/or paper coated with polyethylene.

In some embodiments, a non-active surface of a patch and/or adapter and/or a ventral surface thereof and/or a ventral surface of a control device and/or a frame of a control device may be attached to skin using nonwoven tape for example 3M 1776. For example, a non-active surface of a patch and/or adapter for example a dorsal surface thereof may be attached to a control device by single and/or double sided adhesive, for example 3M 1522. In some embodiments, a substrate (for example a patch and/or an adaptor) may be configured for connection to a control device. Optionally, a surface and/or an edge of a mobile portion of the substrate may be configured for attachment to the device and/or an actuator thereof. Optionally, a surface and/or an edge of a stationary portion of the substrate may be configured for attachment to the device. For example, a part of a dorsal face of the patch may be connected to the control device. Alternatively or additionally an edge of the patch may be connected to the control device. For example, the attachment may be by means of an adhesive and/or a magnet and/or a clip and/or a hook and/or a slit.

In some embodiments, the mobile portion of the substrate will be configured to attach to an actuator and/or mobile portion of the device and/or be engageable and/or disengageable to the skin of a subject. Optionally the stationary portion of the substrate will be configured to attach to an immobile portion of the device and/or a frame of the device and/or be stationary with respect to the skin of a subject (for example remain engaged and/or disengaged as long as the device is attached).

Some embodiments of the system of the invention may be advantageous in "chronotherapy", in which drug delivery is timed in accordance with a body rhythm, for example a circadian rhythm and/or a rhythm of a disease. In some embodiments, chronotherapy, may increase therapeutic efficacy and/or reduce side effects in comparison to constant delivery. Optionally, dose control may be programmed to deliver redefined doses that coincide with peak disease symptoms. For example, this may be useful when symptoms peak at night while asleep or immediately upon waking.

Circadian rhythms may be described as physical, mental and behavioral changes that follow a roughly 24-hour cycle and/or respond to light and darkness in an organism's environment. Circadian rhythms may is some cases influence sleep-wake cycles, hormone release, body temperature and other important bodily functions. They have been linked to various sleep disorders, such as insomnia. Abnormal circadian rhythms have also been associated with obesity, diabetes, depression, bipolar disorder, seasonal affective disorders, asthma attacks, coronary infarction, angina pectoris, stroke and ventricular tachycardia, among others.

In some embodiments, a control system will not interfere with the active portion and/or action of a drug patch. For example, the device may not interfere with the chemical composition and/or physical properties of some, any or all of the patch reservoir and/or the adhesive and/or the release liner. Optionally changes will occur in aspects of the active portion of the patch due to exposure or protection from contact with skin. For example by removing the active surface from the skin of a subject and/or protecting the active surface, chemicals that would have diffused out of the patch may remain and/or build up and/or change concentration. In some embodiments, the dosage control system does not compromise the active envelope and/or stack of the patch either with energy, chemical and/or physical means. In some embodiments, the dosage control system does not add any intervening substance between the active surface of the patch and the skin of a subject.

In some embodiments a dosage control system may work with a conventional drug patch. For example the drug patch may have a predetermined delivery behavior. Optionally the predetermined delivery behavior may depend on characteristics of the subject and/or his skin. Optionally the active surface of the patch will be substantially uniform over 70% and/or 90% of its surface. For example the patch may not include control regions and/or programmable components and/or a sensor.

In some embodiments, the present invention may be used to facilitate transdermal drug administration of any drug including for example Clonidine, Diclofenac Epolamine, Estradiol, Levonorgestrel, Norethindrone Acetate, Norelgestromin, Ethinyl Estradiol, Fentanyl, Lidocaine, Tetracaine, Methylphenidate, Nitroglycerin, Amlexanox, Oxybutynin, Rifastigmine, Scopolamine, Selegiline, Testosterone, Nicotine, Methyl Salicylate, Menthol, Epinephrine, Rotigotine and/or any combination thereof.

In some embodiments, the present invention may be used to facilitate drug administration with an existing transdermal system, for example Catapres TTS, Flector, Vivelle, Climara, Vivelle-Dot, Alora, Menostar, Estraderm, Climara Pro, Combipatch, Ortho Evra, Duragesic, Lidocanine, Synera, Daytrana, Nitro-Dur, Minitran, Oradisc A, Oxytrol, Exelon Patch, Transderm-Scop, Emsam, Androderm, Nicoderm, Habitrol, Prostep, Salonpas.

Some embodiments of a system according to the current invention may be used to transdermally deliver an active drug (for example propranolol, nifedipine, verapamil, enalapril, isosorbide 5-mononitrate and digoxin, an anti-asthmatic (e.g. theophylline and terbutaline), an anticancer drug, a psychotropic drug, an analgesic, a local anesthetics and/or an antibiotic).

### DETAILED EMBODIMENTS

Before explaining at least one embodiment of the invention in detail, it is to be understood that the invention is not necessarily limited in its application to the details of construction and the arrangement of the components and/or methods set forth in the following description and/or illustrated in the drawings and/or the Examples. The invention is capable of other embodiments or of being practiced or carried out in various ways.

### Delayed engagement of an active surface of a medicine patch

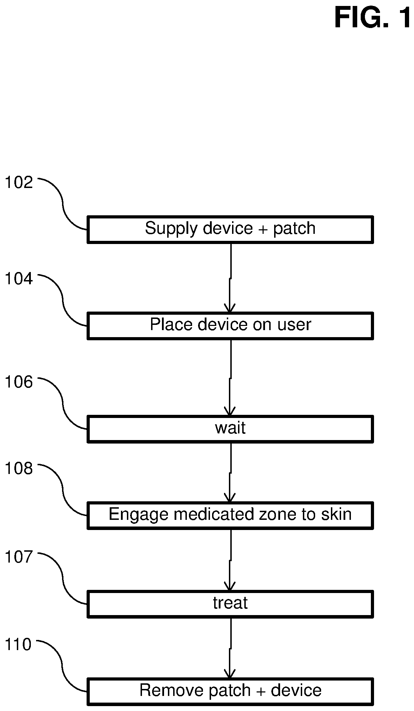

Referring now to the drawings, *FIG. 1* is a flow chart illustrating a method of delayed engagement of an active surface of a drug patch in accordance with some embodiments of the present invention. In some embodiments, a device is supplied **102** with a drug patch stored **104** in the device. Optionally the device is placed **104** on a user's skin. The patched is then engaged **108** to the user's skin after a delay **106.** For example the patch may be engaged **108** by the device according to a preprogrammed time and/or in response to a command and/or in response to an event. When the patch is engaged **108** to the skin of the subject, it optionally treats **107** a condition for example by releasing a drug and/or preparing the skin and/or other treatment. For example that patch may treat the epidermis to improve the drug delivery. Optionally at the end of treatment **107** the device and/or patch may be removed **110** from the subject. Alternatively or additionally the device may disengage the patch and/or engage more active area of the patch (for example as described herein in further embodiments). Alternatively or additionally, the system may be designed to release a measured dose and/or remain active for a limited time and/or cease and/or switch without external intervention.

In some embodiments, a device may be supplied preloaded with a patch from a supplier (for example a drug producer and/or a manufacturer). Optionally the patch may be supplied in a stored state and/or ready to use. For example a user may receive a ready device and/or may place the device on his skin and/or activate the device. Alternatively or additionally, a device may need to be primed before use. For example, a protective liner may be exposed. For example, before placement **104** the liner may be removed by a user. Optionally before placement **104** of the device on a subject, the patch may be moved to a storage position. For example the patch may move to the storage position automatically upon opening a storage container (for example a blister) and/or in response to another step in priming of the device (for example in response removing a protective liner) and/or by a command.

In some embodiments, a patch may be supplied separate from the engagement device. For example, a patch may be included in package with the device and/or supplied entirely separately from the device. Optionally the patch may be packaged in a protective package.

In some embodiments, a patch may be designed for manual placement on the skin without the engagement device, for example a conventional patch. Optionally, an interface may be supplied to connect the patch to the device. Alternatively or additionally, the patch may be connected directly to the device. Alternatively or additionally, a patch may be designed and/or packaged for use with the device. Optionally, the user may remove the patch from a protective wrapper and/or load the patch to the device. Alternatively or additionally, a patch may be an integral part of a cartridge and/or device. For example, the cartridge may be disposable and/or single use while the controller and/or driver may be reused. Alternatively or additionally, the entire device may be disposed of for example with a used patch.

In some embodiments, the device may engage **108** the patch to the subject (for example by putting an active portion of the patch in contact with the skin of the subject). Optionally, engagement **108** may occur after a time delay **106.** For example, time delay **106** may be preprogrammed. Alternatively or additionally, engagement **108** may be in response to a command. Alternatively or additionally, engagement **108** may be in response to an event. For example, a sensor may detect a medical condition requiring treatment and the device may respond by engaging **108** the patch. Alternatively or additionally engagement 108 may be in response to compound trigger (for example a sensor may alert a medical professional who may transmit a command to engage **108** the patch and/or a patch may be engaged in response to a certain sensor output only at certain times and/or under certain conditions [for example the patch may be engaged only when a cumulative dosage in a determined time period is with a specific range]).

In some embodiments a patch engaging device may include a logical controller, for example a processor. Optionally the processor will give commands to engage **108** the patch according to preprogrammed instructions and/or received data and/or received commands. Alternatively or additionally a device may progressively meter out a patch having one or more active surfaces. When an active surface is engaged **108** it may perform a treatment **107** for a predetermined period and/or until it is disengaged for example by being removed **110.** Optionally, the device may perform the disengagement of the patch (for example as described in various embodiments herein).

In some embodiments, a delayed patch engaging system may be useful for convenience and/or safety. For example, when a subject is to receive a drug at night, a device may engage a patch at a predetermined time without needing to depend on the subject waking up and remembering the drug. In some embodiments, the machine may engage the patch without disturbing the subject's sleep. Alternatively or additionally, the device may be used to engage a patch to a subject who has limited ability of self administration. For example a patch may be engaged to a physically disabled subject and/or a mentally limited and/or senile subject without requiring the presence of a caretaker, for example at an inconvenient time. Alternatively or additionally, a device may be used by a veterinarian to engage a patch to an animal at a prescribed time without needing the veterinarian to be present. Alternatively or additionally, the device may be used to apply a dose to subject under monitoring whenever and/or wherever an acute condition occurs. Alternatively or additionally, a delayed engagement device may be used to maintain a determined dosage. For example as a patch is depleted, the device may progressively engage more of the patch, maintaining a constant dosage and/or the speed of engaging may be controlled to maintain a desired dosage regime (for example through a preprogrammed engaging regime and/or speeding or slowing engagement in response to a sensor). Alternatively or additionally, the device may be used to increase the level of compliance in environments where compliance is a critical and/or problematic (for example treatment of mental patients and/or chemotherapy and/or drug testing). For example use of a delayed patch engagement device may reduce the requirements of subject supervision and/or associated subject time in a hospital and/or clinic. Alternatively or additionally the device may be used for non-medicinal drugs and treatments. For example, a device may engage a patch with a drug against motion sickness to a sleeping subject a few hours before an early morning flight and/or a device may engage a patch including a stimulant a to a subject shortly before he needs to wake up.

### Automatic disengaging of medicine patch

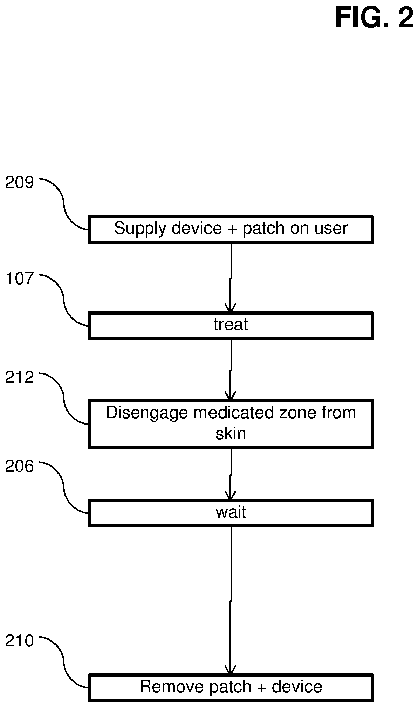

*FIG. 2* is a flow chart illustrating a method of disengaging of a medicine patch in accordance with some embodiments of the present invention. In some embodiments, a device may be used to disengage **212** a drug patch from a subject. For example, a disengagement device may be supplied **209** on a user being treated **107** with a drug patch. Optionally when the treatment should be stopped, the device will disengage **212** the patch from the skin of the subject. Once the patch has been disengaged **212** the device and/or patch may be removed **210** at a convenient time.

In some embodiments, a user may place a device on a subject in an engaged state. The device may leave the patch on during a treatment **107** and then remove the patch. Alternatively or additionally, a machine may be used to perform delayed engagement of a device (for example as described in the previous embodiments) and then to perform disengagement **212.** Optionally the user may load the drug and/or prime the device and/or place the device onto the subject. Alternatively or additionally a user may receive a device preloaded and/or preprimed.

In some embodiments, a device may disengage **212** a patch according to a command and/or in response to a condition (for example in response to a sensor signal) and/or at a preprogrammed time. Optionally, a disengagement time may be programmed into a controller of the device, for example a processor. Alternatively or additionally, the device may progressively remove a patch having active surfaces located at predetermined locations along the patch thereby being removed at a predetermined time. The programming is optionally done by a manufacturer and/or by a user. In some embodiments after disengagement **212** of a patch, the patch and/or the device may be removed **210** and/or disposed of. Optionally, removal of the device may be immediate. Alternatively or additionally, removal may be after a time delay **206.** Alternatively or additionally disengagement **212** of the patch may be reversible. For example, the device may store the disengaged patch and/or engage the patch after storage (for example as described in various embodiments herein).

In some embodiments a patch disengagement device may be used to reduce subject supervision and/or shorten subject time in a hospital or clinic. For example, a subject who would otherwise be required to remain in a clinic to make sure a patch is properly disengaged, may be administered a patch with a device and sent home. For example a patch disengagement device may be used to prevent accidental overdose due to neglecting to remove a patch. For example, a device may remove a patch at a fixed time before an overdose would occur and/or a device may have a sensor that detects initial signs of an overdose and removes that patch before the overdose. Alternatively or additionally, a patch removal device may be under control of a subject and/or may be used to monitor the use of a medicine patch (for example dosage, timing and/or compliance). Alternatively or additionally a patch removal device may be used to remove a patch at an inconvenient time (for example to remove a sleeping drug patch after a subject has gone to sleep and/or to remove a patch from a subject early in the morning without requiring a subject to get up and/or a caretaker to come).

### Controlling of dosage of a medicine patch

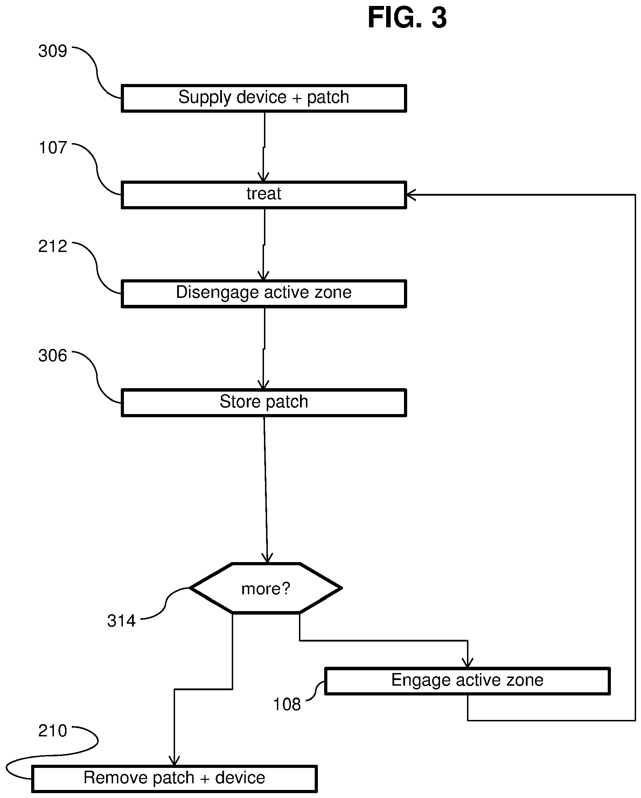

*FIG. 3* is a flow chart illustrating a method of reversibly engaging and disengaging of a medicine patch in accordance with some embodiments of the present invention. In some embodiments, a device may reversibly and/or repeatedly engage and/or disengage a medicine patch. For example, a patch may have an active surface for treatment of a subject. The device may repeatedly disengage **212** the active surface from the subject and/or engage **108** the active surface. Optionally, while the active surface is disengaged, it is stored for re-engaging **108.** Optionally, before and/or during storage, the patch may be protected for example by placing a liner over an active area of the patch while it is being stored **306.** Alternatively or additionally, the patch is stored rolled about itself and/or rolled around a liner or an inflatable mesh. Optionally a patch may be disengaged by blocking contact between the active surface of the patch and the skin.

In some embodiments a patch and or device may be supplied **309** to a subject. Optionally the device may be preloaded. Alternatively or additionally the device may require loading. Optionally the device may be preprimed. Alternatively or additionally the device may require priming. In some embodiments the device will be supplied **309** in an engaged state. For example, treatment **107** may immediately start when the device is placed on the skin of the subject. Alternatively or additionally, the device may be supplied **309** in a disengaged state. For example, treatment **107** and/or engagement **108** of the patch may start after a delay.

In some embodiments, after a period of treatment **107,** a patch may be disengaged **212** and/or stored **306.** During and/or prior to storage **306,** the patch may be protected, for example from contamination and/or depletion. After disengagement, a decision may be made whether to administer more **314** treatment **107.** The decision to administer more **314** treatment **107** may optionally be preprogrammed. For example, a processor in the device may be programmed to continue treatment **107** according to a time schedule and/or dependent on a sensor input and/or based on commands communicated to the processor. Alternatively or additionally, the patch may be formed to perform a particular treatment regime. For example, active and/or inactive surfaces may be distributed along the patch such that the device progressively engages and/or disengages various zones at different times. For example, the device may progressively engage and/or disengage parts of the patch. Optionally, according to the configuration of the patch active and/or inactive surfaces may be engaged and/or disengaged.

In some embodiments a reversible patch engaging/disengaging device may be used to maintain a dosage regime and/or medicine concentration over an extended period. For example, maintaining a dosage may compensate for the dosage rate reduction resulting from aging and/or use of the patch, for example a change of drug concentration in the patch due to depletion. For example a control device may be used to set a pharma kinetic level (PK) in the blood. For example fresh patch may be applied and/or a patch may be applied over fresh of skin. For example, fresh application may stabilize the total transfer in spite of degradation in the skin and/or patch.

### Device for controlling engagement of a medicine patch

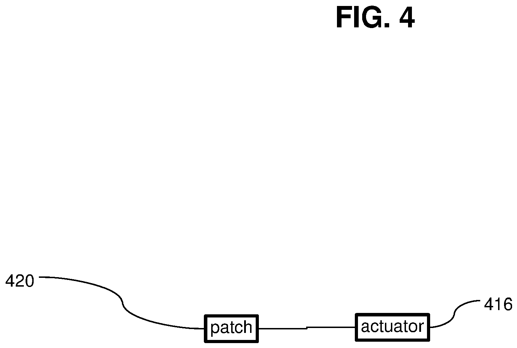

*FIG. 4* is a block diagram illustrating a device for controlling engagement of a drug patch in accordance with an embodiment of the current invention. In some embodiments, a patch controlling device may include an actuator **416.** For example actuator **416** may initiate, increase, decrease and/or interrupt administration of a drug from a patch **420.** Optionally the patch **420** includes a conventional fixed dose drug patch. Alternatively or additionally a patch may be a custom patch. For example, custom patch may include an active area of a conventional patch, for example approved for use for a fixed dosage mounted on a custom substrate and/or custom periphery.

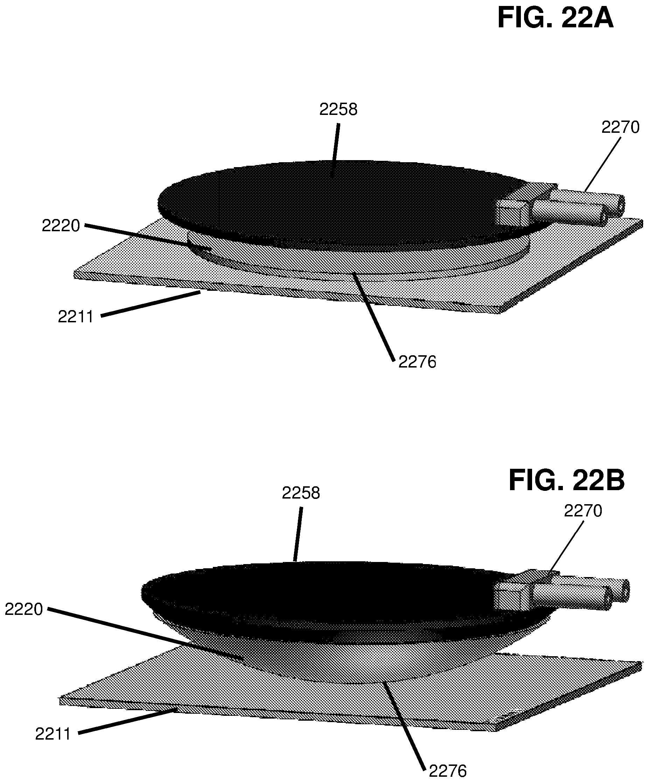

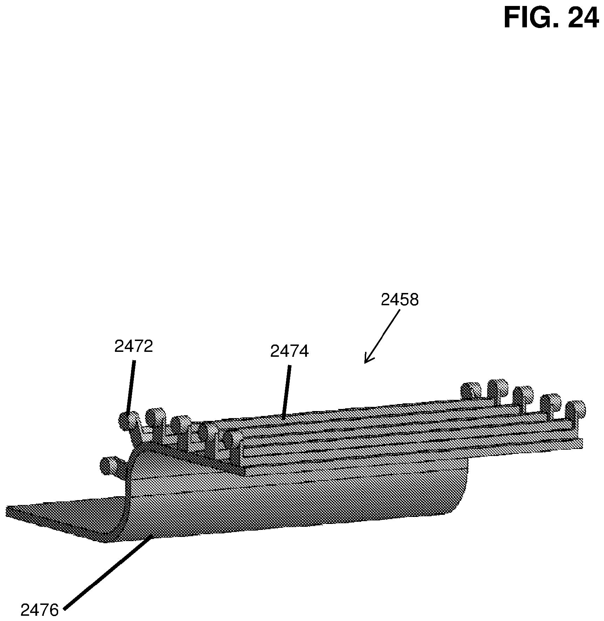

In some embodiments an actuator may be mobile with respect to a frame and/or a subject contact surface of the device. Optionally, actuator **416** may include a roller. For example a roller may roll a patch onto and/or off of a subject. Alternatively or additionally an actuator **416** may include a substrate (for example a flexible belt and/or a flexible strip and/or a ductile actuator [for example that changes shape to engage and/or disengage a portion of a patch for example substrates **2258** and/or **2458** of *FIGs. 22* *and* *24* respectively].

In some embodiment patch **420** may include a single active region and/or multiple active regions. Optionally, different active regions may have the same drug formulation. Alternatively or additionally different active regions may include different drugs and/or formulations.

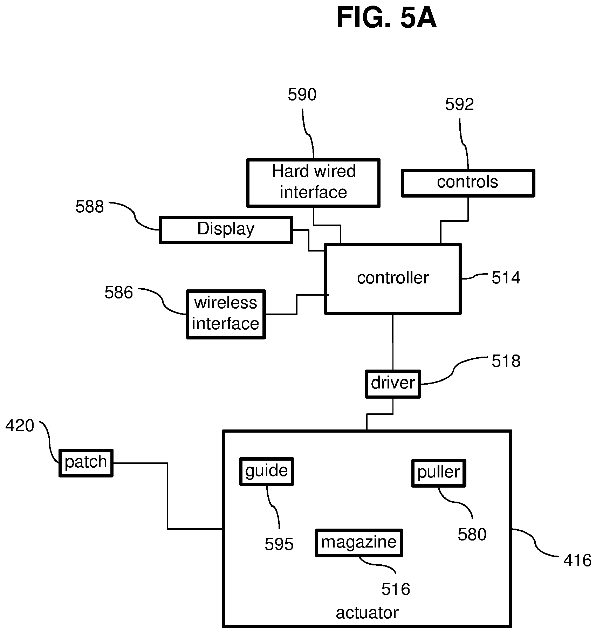

*FIG. 5A* is a block diagram illustrating further optional features of a device for controlling engagement of a drug patch in accordance with an embodiment of the current invention. In some embodiments, a patch controlling device includes a controller **514.** For example controller **514** may be programmable. Optionally controller **514** controls a drive **518** which powers an actuator **416** to switch patch **420** between operation states (for example fully engaged, fully disengaged and/or partially engaged). Optionally actuator **416** may include one or more mechanical sub elements. For example mechanical elements may manipulate patch **420.** For example a guide **595** may direct a separation line between a patch and skin of a subject. Optionally a puller **580** may pull a portion of a patch **420.** For example puller may pull a patch away from skin of a user and/or put tension on patch **420** keeping it connected to guide **595.** Optionally, a patch control device may include a repository **516** for storing a patch.

In some embodiments, a guide may include a roller that rolls along and/or directs a separation line where a patch is separated and/or united with the skin of the subject. Alternatively or additionally a guide may include a sliding object and/or a wiper and/or flattening element and/or a straight edge that directs the separation line.

In some embodiments, a repository **516** may include a roller that stores a patch, for example by rolling it up. Alternatively or additionally a repository **516** may include a region inside a housing and/or a substrate that holds a patch stored away from the skin of the subject.

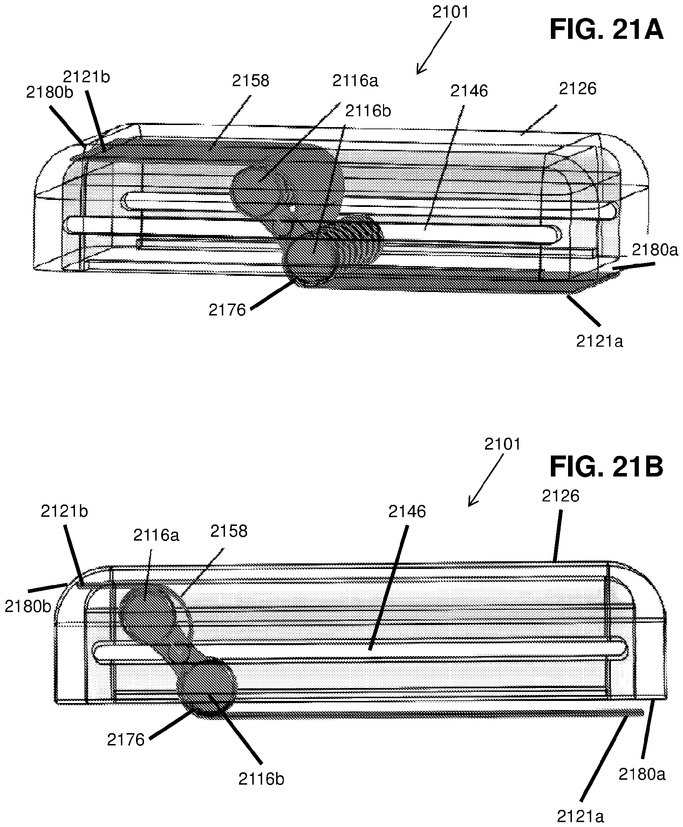

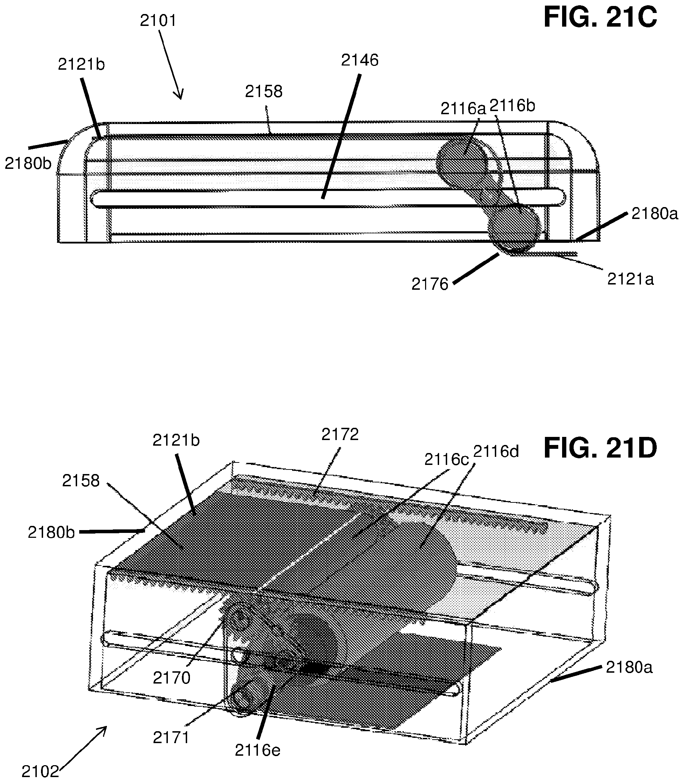

In some embodiments, a puller may include a roller that keeps tension on a patch, for example as illustrated by puller **2116a** of *FIGs. 21A-21C**.* Alternatively or additionally a puller may include an elastic substance and/or a spring and/or a connection to a housing that keeps tension on a patch. Optionally, a puller alone and/or in combination with a guide may pull and/or peel a patch. Optionally a patch may be pulled and/or peeled a peeling angle for example between 0 to 10 degrees from the horizontal and/or between 10 to 45 degrees and/or between 45 to 80 degrees and/or between 80 to 100 degrees and/of between 100 to 135 degrees between 135 to 170 degrees and/or between 170 degrees to 180 degrees where is 0 degrees is pulling the separation line directly towards a separated edge, 90 degrees is pulling the patch directly away from the skin and 180 degrees is pulling the patch directly toward a zone where the patch is adhered to the skin.

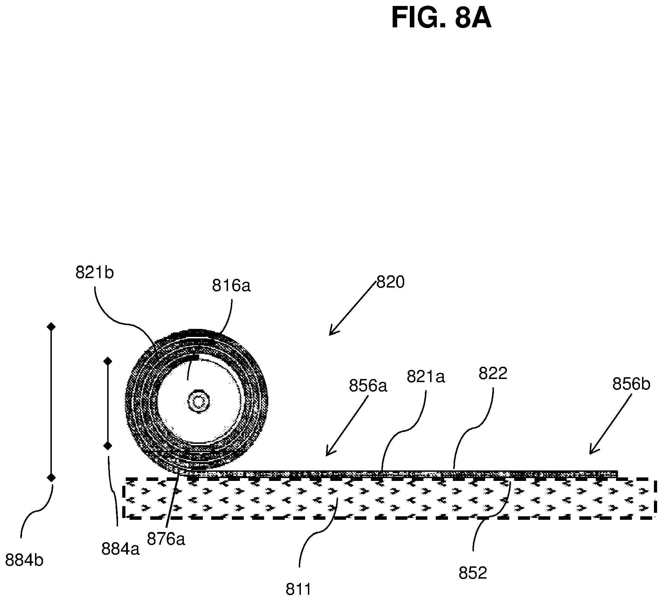

In some embodiment a single component may pull and/or guide a separation line and/or store a portion of the patch. For example when roller **816a** of *FIG. 8A* optionally disengages patch **820,** roller **816a** optionally pulls up on the free end of the patch and/or roller **816a** optionally stores patch **820** by wrapping it around roller **816a** and/or guides separation line **876a.**

In some embodiments, a user interface is provided, for example including a wireless interface **586.** For example a subject may use a personal computing device (for example a smart phone) to communicate with controller **514** over a wireless interface. For example, a cell phone may output status information of the control device and/or a user may use the cell phone as an input device to control and/or program the patch control device. Optionally a patch control device may include a dedicated display **588.** For example display **588** may include a status indicator, for example a LED that shines green when working properly and/or red on a malfunction. Alternatively or additionally display **588** may include a LCD display giving alpha-numeric messages. In some embodiments a patch control device may include a hard wired port **590,** for example a charger port and/or a hard wired communication port and/or a combination port for example an USB port. Alternatively or additionally a patch control device may include local controls **592** for example a button and/or a switch. For example, local controls may include an on/off switch and/or a toggle switch to increase and/or decrease a dosage.

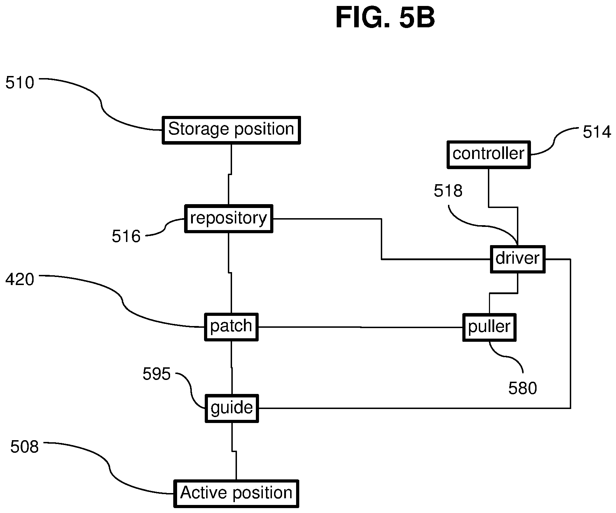

*FIG. 5B* is a block diagram illustrating further optional features of a device for controlling engagement of a drug patch in accordance with an embodiment of the current invention. In some embodiments, a patch controlling device initiates and/or increases a rate of deliver by engaging an active surface of a patch and/or a portion thereof. In some embodiments, a patch controlling device may interrupt and/or reduce a rate of deliver by disengaging an active surface of a patch and/or a portion thereof. An actuator is optionally driven by driver **518.** Optionally driver **518** is controlled by a controller **514.**

In some embodiment, an actuator includes a repository **516** and/or a guide **595** for example to engage a patch by moving an active surface from a storage position **510** to an active position **508** and/or a puller **580.** In some embodiments, a puller **580** disengages patch **420** by moving the active surface from the active position **508** to the storage position. In some embodiments, an actuator may be configured to activate a patch only without deactivating the patch. Alternatively or alternatively an actuator may be configured to deactivate a pre-activated patch. Alternatively or additionally, in some embodiments an actuator may be configured to activate and deactivate patch 420.

In some embodiments, controller **514** may include a processor and/or a communication interface and/or a memory. Optionally controller **514** may receive input from a local and/or remote user and/or a sensor.

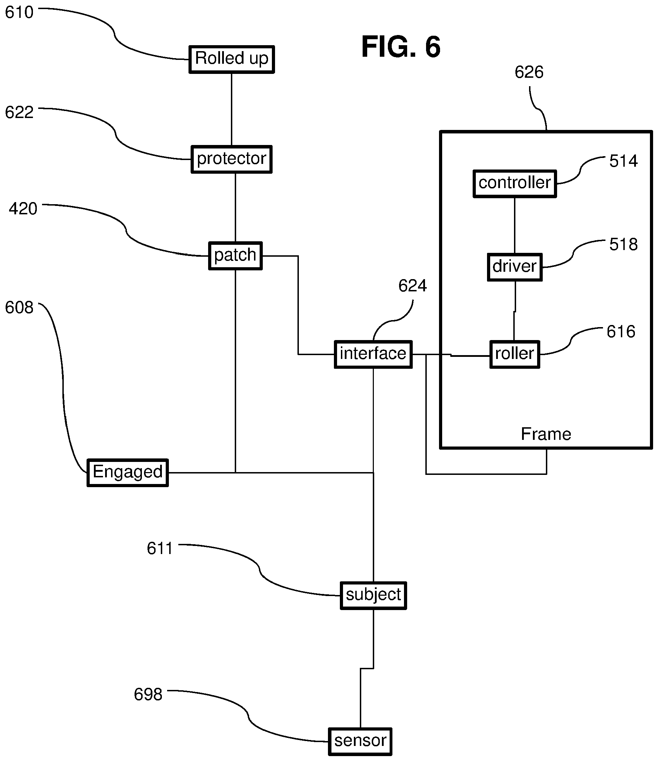

*FIG. 6* is a block diagram illustrating further optional features of a device for controlling engagement of a drug patch in accordance with an embodiment of the current invention. In some embodiments an actuator may include a roller **616.** For example in the stored position, patch **420** may be rolled up onto roller **616.** Optionally a protector **622** to protect a patch in the storage position (e.g. rolled up **610** on a roller). For example, a protector may include a liner separating between layers of patch **420** rolled up on roller **616.**

In some embodiments, an adapter, for example interface **624** may connect between patch **420** and the patch controlling device. For example, interface **624** may include a substrate connecting to patch **420.** Alternatively or additionally a portion of interface **624** may be configured to connect to roller **616** and/or a frame **626** of the patch control device. For example, a mobile portion of the interface may be connected to the roller and/or a stationary portion of interface **624** may be connected to frame **626.**

In some embodiments, driver **518** may reposition and/or rotate roller **616** to move patch **420** from the rolled up **610** stored state to and engaged **608** state contacting a skin **611** of a subject. Optionally, driver **518** may reposition and/or rotate roller **616** to disengage patch from skin **611** and/or roll the patch to the rolled up **610** storage position.

In some embodiments, a patch dosage regulator may be responsive to an event and/or a condition of the subject and/or a sensor output. For example, a sensor 698 may monitor a condition of a subject. Optionally, sensor output may be sent to controller 514. Alternatively or additionally, sensor output may be sent to an external decision maker (e.g. a processor and/or a person) who may send a message to controller 514. Based on the sensor output controller 514 may engage and/or disengage a drug patch and/or increase and/or decrease a dosage. For example sensor 698 may measure one or more of a blood flow, posture, heart rate, a blood oxygen content, breathing rate, temperature.

### Loading a patch to a control device

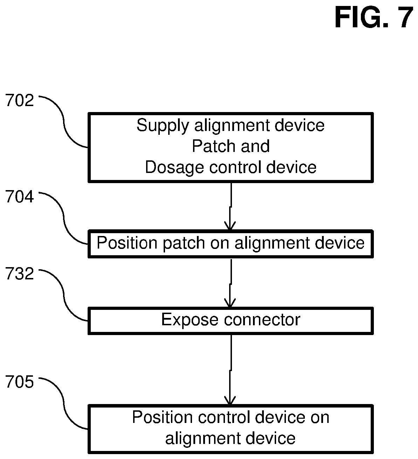

*FIG. 7* is a flow chart illustration of a method of loading a patch to a dosage control device in accordance with an embodiment of the present invention. In some embodiments, an alignment device and a patch and a control device are supplied 702 such that positioning 704 the patch and positioning 705 the control device onto the alignment device loads the patch to the control device.

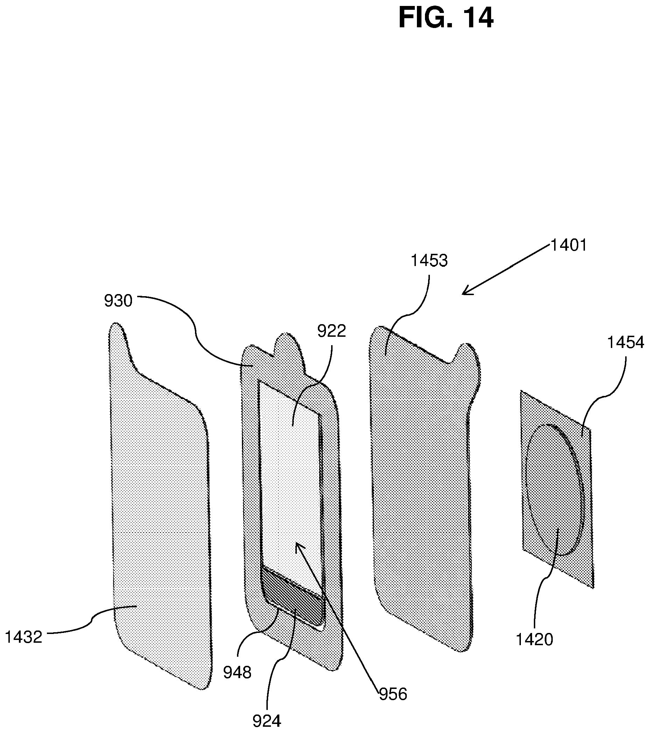

In some embodiments, a patch may be preloaded into an alignment device. Alternatively or additionally a user may place the patch onto the device. For example a user may place the ventral face of patch 920 onto base 939 of device 940. Optionally a user may remove a liner from the patch to expose 732 a connector (For examples removing dorsal liners 1232 from patch 920 and/or liner 1432 of from adapter 1401, for example as illustrated in FIG. 14, exposes 732 connector 924).

### Patch roller

*FIG. 8A* is a perspective side view of a roller in which optionally guides a separation line. A roller optionally also pulls a patch and/or stores a patch in accordance with an arrangement of the disclosure. In some arrangements, patch 820 is stored by rolled up onto a roller 816a. Optionally, roller 816a engages a portion 821a of patch 820 by unrolling portion 821a of the patch along a separation line 876a onto a skin 811 of a subject. For example, in the view of FIG. 8A, roller 816a rolls leftward to engage and/or unrolls patch 820 onto skin 811. Optionally, roller 816a disengages the patch for example by pulling and/or peeling the leading edge of the patch from the skin. For example, in the view of FIG. 8A, roller 816a roller rightward to roll up and/or disengage patch 820 from skin 811.

In some arrangements, the diameter of a rolled up patch 820 may increase as more patch 820 is rolled up. For example, before the patch starts being rolled up, the total diameter may be roller diameter 884a. As the patch is rolled up, the roll diameter 884b may increase. For example, the roller diameter 884a may start at 8mm and roll may grow for example to a roll diameter 884b of about 20mm as the patch is loaded onto roller 816a.

In some arrangements, patch 820 includes an active zone 856a and/or an inactive zone 856b. For example, inactive zone 856b may have an active surface on a ventral face thereof and/or may be on the trailing edge of patch 820. Optionally, when patch 820 is fully rolled up onto roller 816a, only a trailing section including inactive zone 856b is in contact with skin 811 and/or active zone 856a is not in contact with the skin. Optionally, when active zone 856a is not in contact with the skin, no treatment occurs. Alternatively or additionally, different zones of a patch may include different drugs for different treatments. For example, zone 856a may include a first drug that is applied to skin 811 from the beginning of a treatment and zone 856b may have a different drug which is applied to the skin later in the treatment.

In some arrangements, patch 820 includes a skin contact surface 852 on a ventral side thereof. Optionally, a dorsal side of the patch is covered by a protector 822. For example, protector 822 may include a liner. Optionally protector 822 includes a chemically inert and/or clean and/or sterile and/or non-stick surface. Optionally, during storage, for example on a rolled up portion 821b of patch 820, protector 822 covers skin contact surface 852 of active zone 856a of patch 820. For example, roller 816a may peel and/or place patch 820 from and/or to the skin on a separation line 876a. Optionally, separation line 876a is the line of contact. For example at separation line 876a, patch 820 is sandwiched between roller 816a and skin 811.

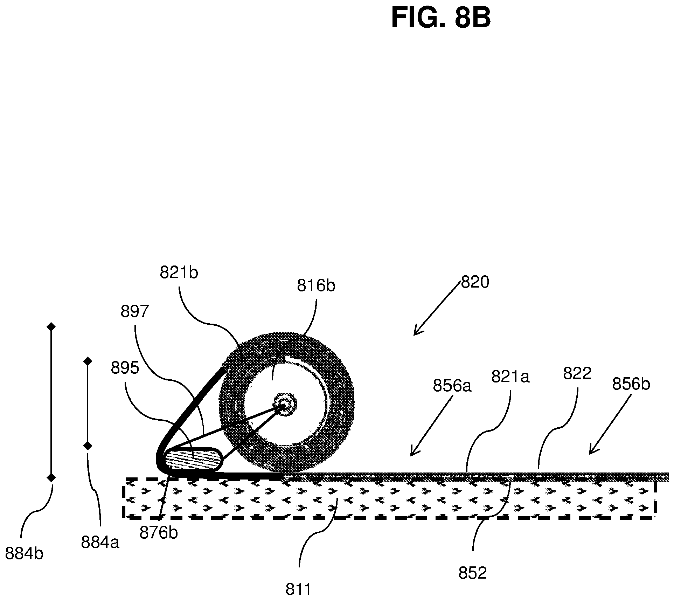

*FIG. 8B* is a perspective side view of a patch control system having a roller 816b which optionally keeps tension on the patch and/or stores the patch and a separate guide 895 which optionally guides a separation line 876b in accordance with an arrangementof the current invention. In some arrangement, guide 895 is supported on supports 897.

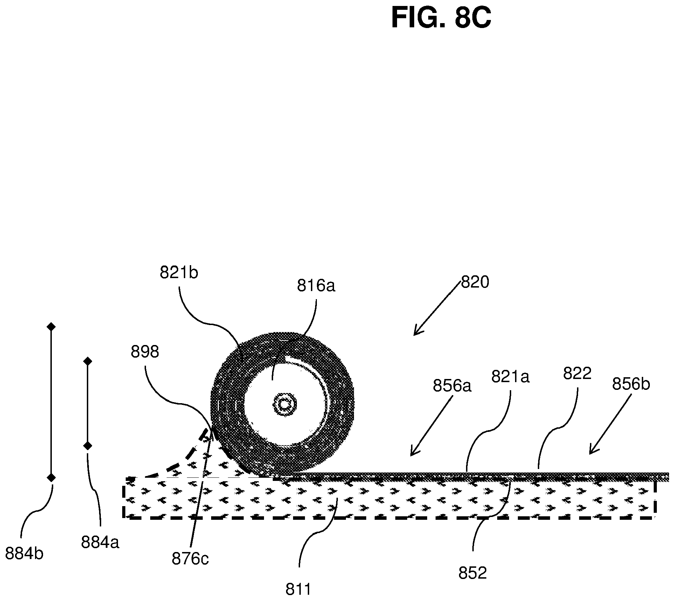

*FIG. 8C* is a perspective side view of a roller 816a which optionally guides a separation line 876c, pulls a patch 820 and/or stores patch 820 in accordance with an arrangement of the disclosure. In some arrangements, patch 820 will deform skin 811, for example by pulling skin 811 into a mound 898 while disengaging patch 820. In some arrangements, the deformation of skin 811 may affect the peeling angle of patch 820. For example by having a smaller guide the effect of mounding of the skin on the angle of peeling may be greater. For example the angle of peeling may be decreased by more mounding of the skin (for example due to a sticker adhesive) and/or the angle of peeling may be decreased by using a larger guide (for example a roller with a larger diameter). The stickiness of the patch, size of the guide and/or direction of pulling may be adjusted for example to reduce discomfort when peeling a patch.

### Exemplary stationary reversible patch roller system

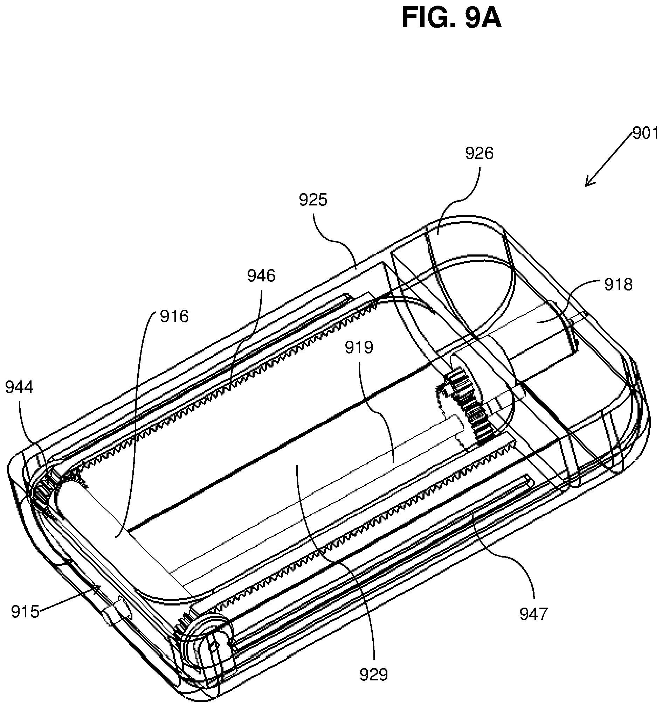

*FIG. 9A* is a perspective view of a ventral side of an exemplary patch control device 901 in accordance with an embodiment of the present invention. Optionally device 901 reversibly engages and/or disengages portions of a patch. Optionally device 901 stores disengaged portions of the patch. In some embodiments, device 901 includes a roller 916. Optionally roller 916 rolls backwards and/or forward inside a frame 926. For example, when roller 916 rolls in one direction it rolls up and/or disengages a portion of a patch. For example, when roller 916 rolls in an opposite direction it unrolls and/or engages a portion of the patch. Optionally, a stored portion may be protected by a liner and/or stored on a roller 916.

In some embodiments, frame 926 includes a shell surrounding roller 916. Optionally frame 926 includes a contact surface 925 on a base of the ventral side of frame 926. Optionally contact surface 925 surrounds an opening 929. For example, contact surface 925 fully surrounds opening 929. Alternatively or additionally a contact surface may only partially surround opening 929. For example, when the device 901 is placed onto a subject, contact surface 925 lies against the skin of the subject. Optionally when a patch is in an engaged state, an active surface of the patch is exposed to and/or contacts the skin of a subject through opening 929. Optionally in a disengaged state, the active surface may be rolled up on roller 916 and does not contact the skin of the user.

In some embodiments, frame 926 includes one or more tracks. For example, frame 926 includes a sliding guide track 947 and/or a friction track 946. Optionally a driver 918 (for example including a DC motor) drives a transmission 919 to pull or push a roller assembly 915 longitudinally along guide track 947. For example transmission **919** may include a threaded shaft which is engaged to a thread in assembly **915.** For example rotating transmission **919** in one direction pulls assembly **915** longitudinally along track **947** towards driver **918.** For example rotating transmission **919** in an opposite direction pushes assembly **915** longitudinally along track **947** away from driver **918.**

In some embodiments, as assembly **915** moves longitudinally along guide track **947,** roller **916** rotates to roll up and/or unroll a patch. Optionally as assembly **915** moves in a first direction roller **916** rolls up and/or disengages a patch. Optionally as assembly **915** moves in an opposite direction roller **916** unrolls and/or engages the patch. For example, as assembly **915** moves longitudinally, a friction contact surface **944** of roller **916** rolls along friction track **946,** causing roller **916** to rotate. For example, friction track **946** may include teeth that engage to teeth of friction contact surface **944** which optionally includes a gear.

In some embodiments a synchronizer may synchronize the rate of engaging of patch **920** with the rate of movement roller **916.** For example the teeth of track **946** may be differentially spaced. For example the spacing of the teeth may be configured to synchronize rolling of roller **916** with its linear movement. Alternatively or additionally a controller may separately control the linear and rotational movements (for example with separate drivers).

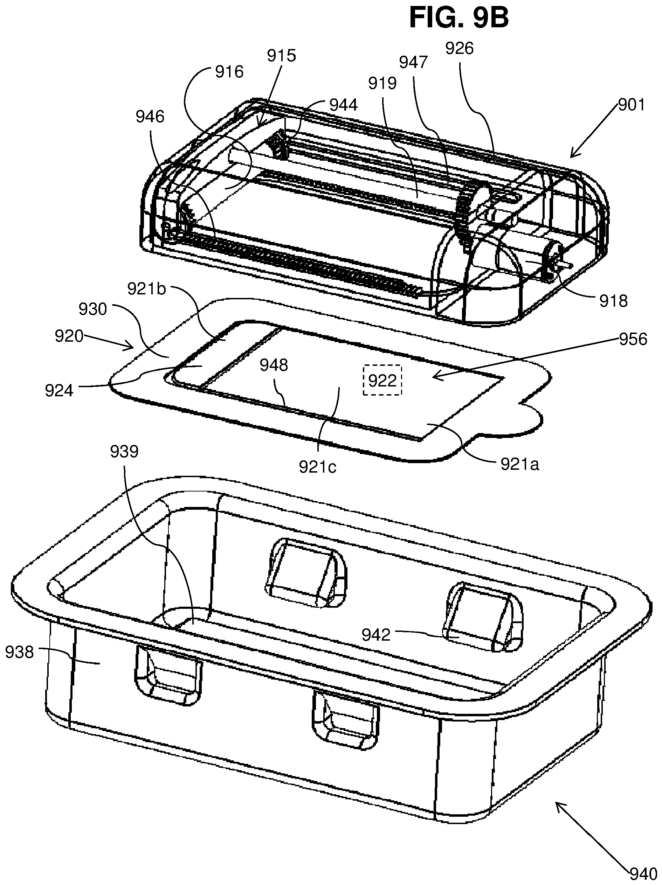

*FIG. 9B* is a perspective view of a dorsal side of a patch control system in accordance with an embodiment of the present invention. Optionally, a patch control system reversibly engages and/or disengages portions of a patch. Optionally, a patch control system may stores disengaged portions of the patch and/or protects stored portions, for example by a liner. For example the portions are stored on a roller. In some embodiments, a patch control system may include a patch control device **901** and/or an optionally custom patch **920** and/or an alignment device **940.**

In some embodiments, patch **920** includes a stationary portion **930.** Optionally, stationary portion **930** may include adhesive of both sides. For example adhesive on the dorsal side may adhere stationary portion **930** to contact surface **925** of frame **926.** For example, adhesive on the ventral side of stationary portion **930** may adhere to stationary portion **930** and/or contact surface **925** of frame **926** to the skin of a subject. For example, when device **901** is positioned on a subject, stationary portion **930** may be immobile and/or sandwiched between contact area **925** of frame **926** and the skin of the subject. In some embodiments, stationary portion **930** surrounds a disengageable portion **956** of the patch (the disengageable portion **956** optionally includes a leading portion **921b,** a trailing portion **921a** and/or a mid portion **921c.**

In some embodiments, connections between patch **920** and device **901** are made by a single simple movement. For example, device **901** has all of its connections to patch **920** exposed on one side (e.g. the ventral side of device **901** connects to the dorsal side of patch **920).** For example, a contact zone of roller **916** which contacts adhesive **924** of the leading mobile end of mobile portion **956** and/or a contact surface **925** of device **901** which connects stationary portion **930** of patch are all exposed on a ventral face of device **901.** For example by pushing device **901** downward onto the dorsal surface of patch **901** the patch is loaded to device **901.** Optionally all of the connection surface lie on a place such that device **901** connects to patch **920** while patch **920** is lying flat on a surface. Alternatively or additionally an alignment device **940** has a base with contours that position patch **920** properly to connect to each part of device **901.** For example, the contact zone of roller **916** may be recessed with respect to contact surface **925.** Optionally the base of alignment device **940** holds the free leading portion **921b** extended upward to contact the contact roller **916** when device **901** is lowered into place onto alignment device **940.** Optionally, after connection, a mobile engageable portion **956** may be wound to roller **916** (e.g. into a disengaged state) before attaching device **901** to a subject. Alternatively or additionally, device **901** may be attached to a subject in an engaged state.

In some embodiments, stationary portion **930** may partially surround engageable portion **956.** For example, stationary portion **930** may just extend from a trailing portion **921a** of engageable portion **956** of patch **920.** Alternatively or additionally a patch may be entirely wound onto a roller without a frame contact area.

In some embodiments, a leading portion **921b** and/or a mid portion **921c** of engageable portion **956** is optionally separated from frame contact area by a discontinuity, for example a cut out **948.** For example, cut out **948** permits leading portion **921b** and or central portion **921c** to roll up onto roller **916** while stationary portion **930** remains stationary with respect to the skin of the subject, for example on or close to the skin of the subject. Alternatively or additionally, cut out **948** permits leading portion **921b** and or central portion **921c** to roll up onto roller **916** while stationary portion **930** remains stationary with respect to frame **926,** for example sandwiched between contact surface **925** of frame **926** and/or the skin of the subject.

In some embodiments, patch **920** may be sold as a whole conventional patch without a discontinuity. For example the discontinuity is optionally cut into the patch after production and/or sale. Optionally, a device and/or die may be supplied to cut a discontinuity. For example, the cut out device may be a separate device, and/or may be including in packaging of a patch and/or of a dosage control device. For example alignment device **940** may include a die and/or blade to cut the discontinuity. For example a convention patch may be modified by cutting a discontinuity. Optionally the discontinuity will be in a non-active area. For example cutting a discontinuity into a patch may not compromise an active area and/or a drug reservoir and/or a release membrane.

In some embodiments, leading portion **921b** of disengageable portion **956** of patch **920** includes adhesive **924** on its dorsal side. Adhesive **924** optionally connects leading portion **921b** to roller **916.** Alternatively or additionally, roller **916** may be coated with adhesive. Alternatively or additionally, roller **916** and/or leading portion **921b** may include a magnet and/or a clip and/or a hook and/or a slit to for example for connecting the free portion of the patch to the roller.

In some embodiments, a protector may protect an active zone and/or a skin contact surface of a patch during storage. For example, a flexible backing including for example a liner **922** covers mid section **921c** of stationary portion **930.** When patch **920** is stored (for example by rolling up onto roller **916** such that engageable portion **956** is wound around roller **916)** the skin contact surface of engageable portion **956** is optionally covered by liner **922.** Optionally liner **922** is made of a chemically inert and/or non-stick and/or non-reactive substance that will protect the drug in engageable portion **956.** Optionally liner **922** is made of a non-stick substance that will protect the adhesive on the skin contact area of engageable portion **956** of patch **920.** For example liner **922** may be made of a non-reactive coating over a substrate. For example a non-reactive coating may include Polyethylene and/or the substrate may include a metal foil (e.g. aluminum) with a thickness between 0.05 and 0.2 mm. Alternatively or additionally a substrate may include paper and/or plastic.

In some embodiments, a patch control system may include an alignment device **940.** For example alignment device **940** may include a cavity of a blister package for patch **920** and/or device **901.** For example, device **901** and/or patch **920** may be packed inside of device **940** and sealed with a backing for shipping and distribution. Device **940** optionally includes interference elements **942.** For example interference elements may be used to snap device **901** into device **940.** For example elements **942** may hold the ventral face of device **901** firmly against base **939** of device **940.** Optionally device **940** may be used to mount patch **920** onto device **901.** Alternatively or additionally, alignment may be facilitated with printed signs, pins, indentations and/or protrusions for the patch and/or device etc. Alternatively or additionally, device **901** may be preloaded with patch **920.** Alternatively or additionally, patch **920** may be mounted to device **901** without an alignment device. Alternatively or additionally, device **901** may be used with a standard patch and an interface (for example patch **1420** and/or adapter **1401** as illustrated in *FIG. 14*). Alternatively or additionally other options for patch geometry may be used with device **901.**

In some embodiments, positioning the patch and the control device onto the alignment device will connect the patch to the control device. For example, once patch **920** is lying on base **939** with connector **924** exposed, device **901** is placed onto alignment device **940** with contact surface **925** facing towards the exposed connector **924** on patch **920.** Optionally device **901** is pushed past interference elements **942** which snap down on device **901** securely pushing contact surface **925** against stationary portion **930** and/or pushing roller **916** against connector **924.** Optionally alignment device **940** is shaped such that patch **920** and/or device **901** are pushed into proper alignment when they are positioned on device **940.** For example, a cavity of device **940** may have beveled walls **938** leading to a tight fitting compartment such that putting a patch and/or control device into the cavity pushes them into mutual alignment.

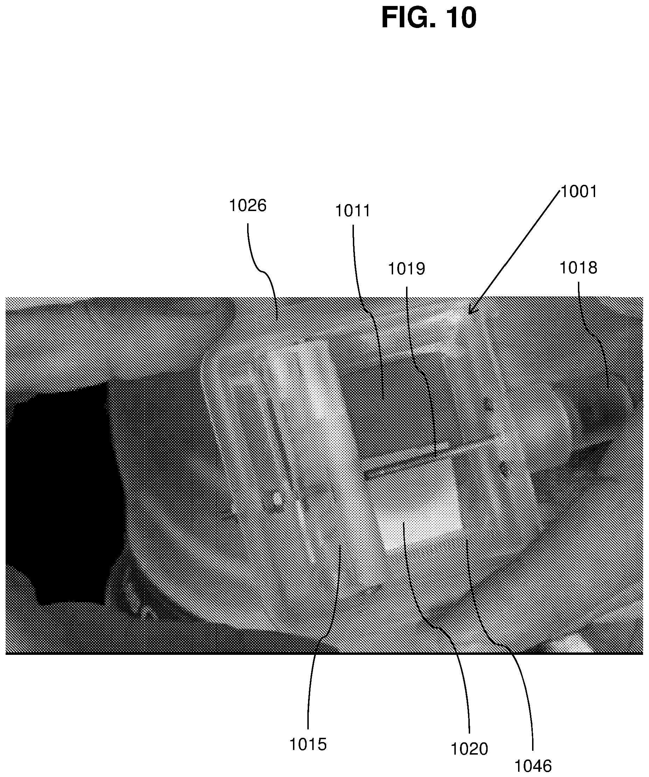

*FIG. 10* is a photograph of an exemplary patch control device **1001** in use in accordance with an embodiment of the current invention. A patch **1020** is optionally loaded into device **1001.** Patch **1020** and device **1001** are then optionally placed against the skin **1011** of a subject. For example, during placement, patch **1020** may be in an engaged state. For example, medicine delivery may begin immediately when patch **1020** and control device **1001** are placed on skin **1011.** Optionally a roller assembly **1015** is pulled by a transmission 1019 toward a driver 1018 to reversibly disengage patchl020 from skin 1011. Optionally, as roller assembly moves towards driver 1018, a roller rolls along a track 1046 rolling up patch 1020. In some embodiments, roller assembly 1015 is pushed by transmission 1019 away from a driver 1018 to reversibly engage patch 1020 to skin 1011. Optionally, as roller assembly 1015 moves away from driver 1018, the roller 1026 rolls along a track 1046 unrolling patch 1020.

In some embodiments, patch control device 1001 may include a controller for example to control driver 918 and/or engagement and/or disengagement of the patch. Alternatively or additionally device 1001 may include a sensor. For example, patch control device 901 may respond to signals from the sensor to engage and/or disengage a patch fully and/or partially. Optionally the sensor output may be sent to a controller of the device.

### Exemplary patch

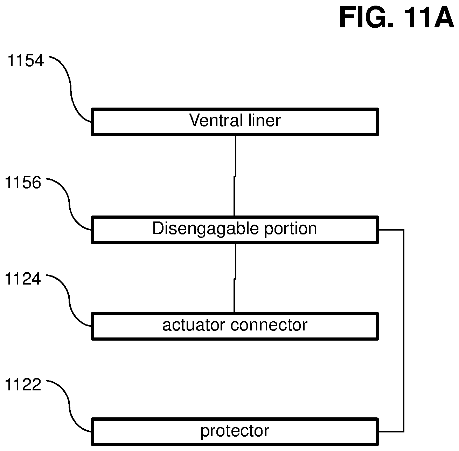

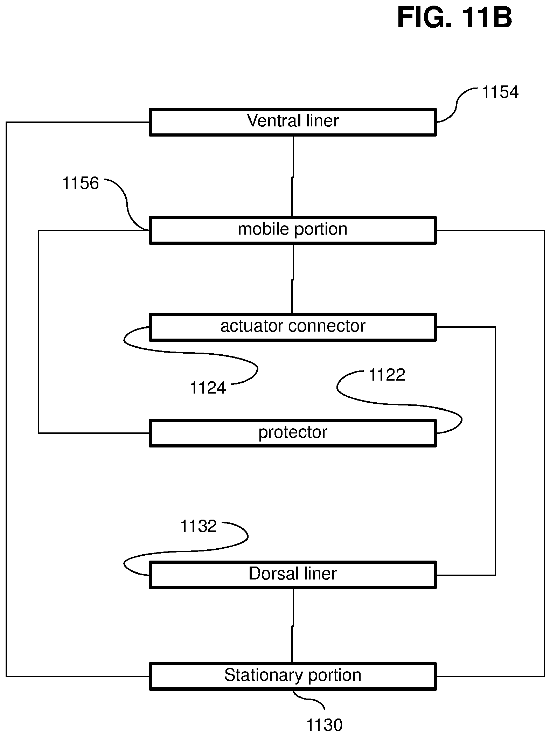

*FIG. 11*A is a block diagram illustration of a simple patch in accordance with some arrangements of the current invention. Optionally, a patch may include a disengageable portion 1156. Optionally, the entire patch may be disengageable from the subject. In some arrangements, the patch includes an active surface. For example the active surface may be on a ventral face of the patch. Optionally the disengageable portion includes a connector 1124 for connecting to an actuator of a control device. The patch optionally includes a protector 1122. For example protector 1122 may protect the active surface during storage by a patch control device. Optionally the patch includes a ventral liner 1154. For example, ventral liner 1154 may protect an active region of the patch before use. For example, the ventral liner may be removed by a user prior to use of the patch. Optionally a patch may include a dorsal liner. For example connector 1124 may include an adhesive surface which is optionally protected by a dorsal liner.

In some arrangements an active surface of a patch may include a drug (for example a medicine patch). Alternatively or additionally the active surface may include an adhesive (for example a medicine patch and/or an interface for a medicine patch).

In some arrangements, connector 1124 may be on a leading edge of the patch and/or on a dorsal face of the patch for example on a dorsal face of a leading portion of the patch.

*FIG. 11B* is a block diagram illustration of a two part patch in accordance with some arrangements of the disclosure. In some arrangements, a patch may include a stationary portion 1130 and/or a disengageable portion 1156. For example the disengageable portion 1156 is configured to be connected by a connector 1124 to an actuator of a patch control device. Optionally stationary portion 1130 is configured to be connected to a stationary portion (for example a frame) of the patch control device. Optionally the stationary portion may include an adhesive on two sides and/or be connected to a dorsal liner 1132 and/or a ventral liner 1154. Dorsal liner 1154 optionally protects actuator connector 1124 of the disengageable portion 1156 of the patch.

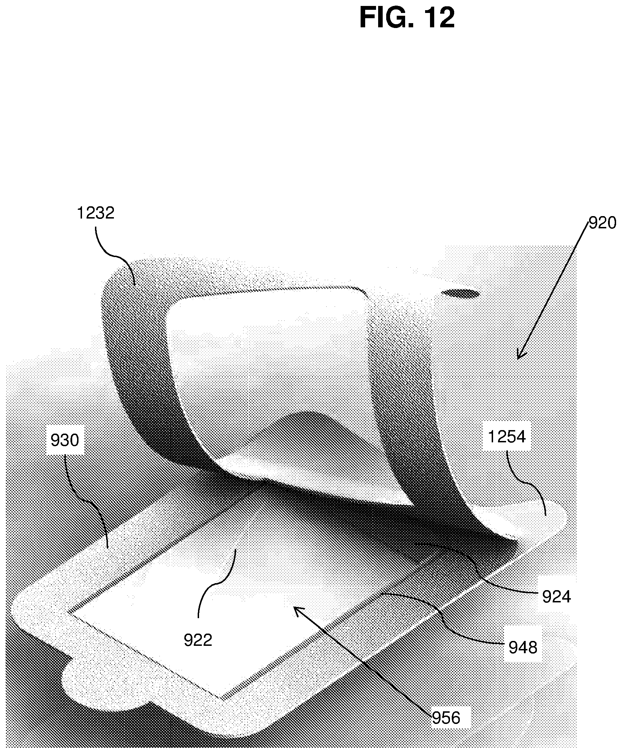

*FIG. 12* is a perspective illustration of a dorsal side of patch 920 in accordance with an arrangement of the disclosure. A dorsal liner 1232 is illustrated partially removed from patch 920. Patch 920 is supplied to a user with, dorsal liner covering an adhesive dorsal surface of stationary portion 930 and/or an adhesive connector 924 for connecting a leading portion of disengageable portion 956 of patch 920 to an actuator of a patch control device. Optionally liner 1232 does not cover protector 922. Alternatively or additional, a dorsal liner may cover a protector for example as illustrated in FIG. 14 where a dorsal liner 1432 covers an entire dorsal face including a protector 922 of a interface patch 1401. In some arrangements, a ventral liner 1453 covers a ventral face of a patch. For example ventral liner 1453 may be peeled off before the patch is placed on the skin of a subject.

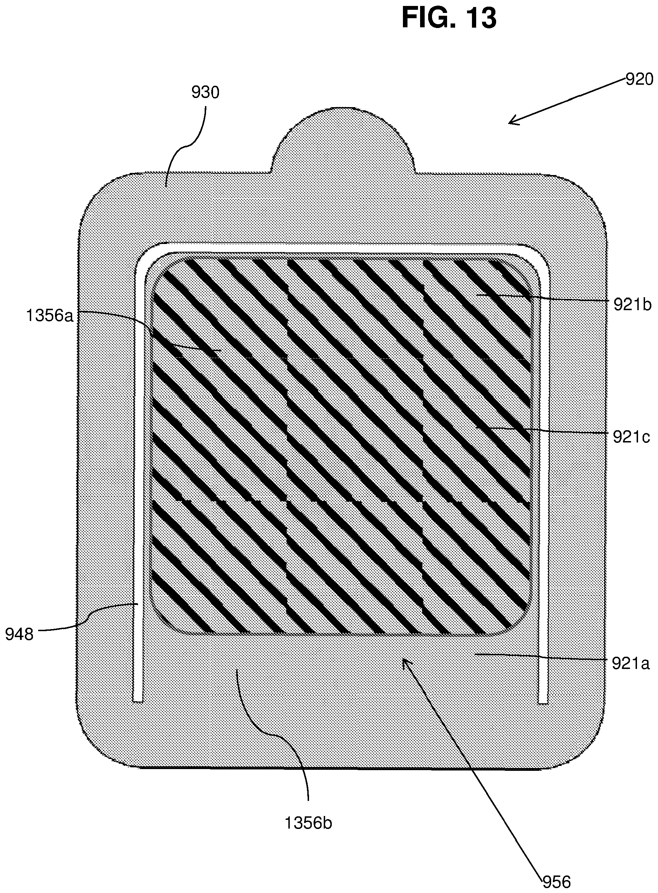

*FIG. 13* is a ventral view of a patch in accordance with an arrangement of the disclosure. In some arrangements a ventral face of patch 920 includes an active surface 1356a on a disengageable portion 956. For example active surface 1356a may include a drug that is absorbed by the skin of a subject when zone 1356a contacts the skin. Optionally a patch control device (for example device 901) controls the magnitude of the surface area of zone 1356a exposed to the skin and/or the rate of absorption of the drug to the subject. For example, disengaging part of zone 1356a reduces the dosage and/or rate of application of the drug. For example, engaging part of zone 1356a increases the dosage and/or rate of application of the drug.

In some arrangement trailing portion 921a of portion 956 may be included in an inactive surface 1356b. For example, misalignment of a device and/or a patch and/or irregularities of the surface of the skin may cause uncertainty in the state of engagement and/or disengagement of the trailing portion 921a and/or the outermost layer of the rolled up patch. Including portion 921a in inactive portion 1356b may decrease the uncertainty of dosage and/or avoid a danger that medicine will continue to be absorbed when patch 920 is supposed to be fully disengaged.

### Exemplary interface for patch

FIG. 14 is a schematic illustration of an interface for connecting a patch to a patch control device in accordance with an arrangement of the disclosure. In some arrangements, an interface may be used to facilitate loading a patch to a patch control device. Optionally an interface, for example, adapter 1401 may be used to facilitate loading a conventional medicine patch (for example patch 1420) to a control device, for example device 901. For example, a dorsal side of adapter 1401 may be configured for loading to device 901 and/or a ventral side of the patch may be configured for attaching to a convention patch.

In some arrangements, a ventral side of adapter 1401 may include a connector 924 and/or a protector 922. Optionally the ventral face of adapter 1401 is covered by a liner 1453 prior to use. Optionally adapter 1401 includes a stationary portion 930 which surrounds and/or partially surround and/or is partially attached to a disengageable portion 956. For example a cut out 948 may separate between some parts of the stationary portion 930 and disengageable portion 956.

In some arrangements, the ventral face of disengageable portion 956 of adapter 1401 includes a substrate and/or an adhesive. Optionally the dorsal face of patch 1420 is adhered to the ventral face of disengageable portion 956 of adapter 1401. The patch 1420 becomes the active region of disengageable portion 956. For example by engaging and/or disengaging the ventral side of portion 956 to the skin of a subject, patch 1420 is engaged and/or disengaged. The combined patch 1420 and adapter 1401 are optionally loaded to device 901 according to the method described in FIG. 7. In some arrangements, a skin contact area and/or an active face and/or ventral face and/or an active surface of patch 1420 will be protected by a liner 1454 prior to use.

In some arrangements, the adhesive area on the ventral said of the adapter 1401 will be larger than the dorsal side of patch 1420. Optionally the adhesive on the ventral side of patch 1401 may be formulated to stick fast to the dorsal face of patch 1420 and/or to be biocompatible and/or to be easily removed from skin. Alternatively or additionally, patch 1420 may be attached to adapter 1401 (for example to the ventral face of portion 956) by ultrasonic welding and/or heat and/or by means of a vacuum. For example, the ventral face of portion 956 may be a substrate that can include attached to patch 1420.

In some arrangements, an adapter and/or an alignment device will be shaped and sized for a particular patch. For example, an area of adhesive on a ventral side of the adapter may fit the patch. For example the adhesive area may be within 1 mm of the dimensions of the dorsal face of the patch and/or within between 1 and 5 mm and/or between 5 and 20 mm. Optionally an alignment device may facilitate fitting the convention patch to the adaptor and/or the patch control device and/or fitting the adaptor to the patch control device. Optionally, attaching patch 1420 to adapter 1401 and/or device 901 does not change an active surface of and/or drug administration property of patch 1420.

Stationary **reversible patch roller system**

*FIGS. 15A-15C* are perspective views of a direct drive patch control device in accordance with the present invention. The control device optionally reversibly engages and/or disengages portions of a patch. Optionally disengaged portions are stored on a roller.

The patch control device 1501 includes a roller 1516 that rotates and slides along a track 947 inside a frame 1526 to engage and/or disengage a medicine patch. Optionally in the disengaged state the patch is stored wound around roller 916.

In some embodiments, roller 1516 is driven by direct drive driver 1518. Driver 1518 optionally rotates a transmission 1519 which rotates roller 1516 and friction wheels 1544. Friction wheels 1544 roll along track while roller 1516 rotates rolling up and/or rolling out the patch. In some embodiments, device 1501 is loaded uses the same patch and/or is loaded as described above with respect to device 901.

In some embodiments, roller 1516 may rotate and/or move linearly along track 947. For example, propulsion may be provided by a friction wheel 1544 (e.g. a gear) rolling along a friction track 946 (e.g., a toothed track) formed in housing 1526. Driver **1518** optionally turns roller 1516. For example driver **1518** may be located inside the roller 1516. This configuration may save space over an external driver.

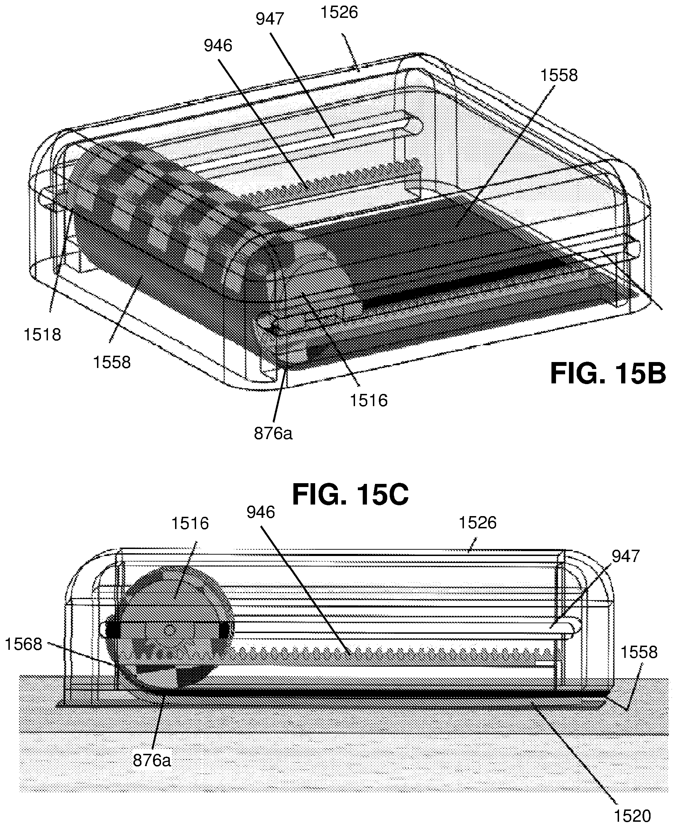

Optionally, the rotating speed of roller **1516** is adapted to synchronize the speed of the surface of the substrate to the linear rate of uptake and/or release of the patch. For example as the diameter of the roll grows (e.g. with increasing numbers of layers of substrate and/or patch over roller 1516) the rotation rate may be reduced with respect to the rate of linear movement of roller 1516. Optionally a synchronizer may synchronize the rate of engaging of uptake or output of substrate **1558** with the rate of movement roller **1516** and/or separation line 876a. For example distance between teeth in the track 946 may be adjusted synchronize linear motion and rotation of roller 1516. For example in *FIG. 15B**,* where the diameter of the roll increases as roller **1516** moves rightward, the distance between the teeth in track 946 may get larger as one moves down the track from left to right and/or the ratio of rotation to linear movement is reduced as roller **1516** moves from left to right. In some embodiments, synchronizing with the roller linear and rotational velocity may to facilitate deployment of the patch with reduced wrinkle or skin pinching and/or hair pulling. Alternatively or additionally a controller may separately control the linear and rotational movements (for example with separate drivers).

*FIGS. 15C* illustrate an optional way of mounting a standard drug patch **1520** onto roller **1516.** A substrate **1558** (for example an adhesive foil without the substance on the patch) is optionally connected to the roller **1516** and/or extends out from the roller **1516** over the bottom of housing **1526.** In order to connect the patch **1520** to the roller, housing **1526** is optionally placed onto patch **1520,** whereupon the patch **1520** sticks to substrate **1558.** For example, roller **1516** may peel and/or place substrate **1558** to from and/or to the skin on a separation line **876a.**

In some embodiments, once patch **1520** is attached to substrate **1558,** a liner may be removed from the active face (e.g. the bottom face in *FIG. 15C*) of the patch **1520.** The exposed patch **1520** and/or device **1501** are then placed on the skin in an engaged state. Alternatively or additionally, before placing device **1501** on the skin, driver **1518** may turn the roller **1516** to cause patch **1520** and/or substrate **1558** to wrap and/or roll around the roller **1518** into a disengaged and/or stored state. In some embodiments, a protective element (e.g. to protect a patch during storage) is a part of the control device. For example the back of substrate 1558 optionally serves as a liner and/or protective layer on the patch 1520 (e.g. the active surface thereof) during storage. Optionally, device 1501 is placed onto a subject in the disengaged state. Alternatively, instead of using driver 1518 to wind up patch 1520, housing 1526 may be moved across a surface (e.g., assembly table) to cause roller 1516 to turn and move in track 947. Optionally, a tongue 1568 is provided near the roller 1516 just before patch 1520 begins to wrap around the roller 1516. For example, tongue 1568 may catch and lift off any liner that was mounted on patch 1520, so that patch 1520 rolls up on roller 1516 without the liner.

In some embodiments a roller (for example roller 916 and/or roller 1516) may be driven by a pulley and/or a linear actuator and/or a spring drive.

### A rotating device with a continuous track substrate

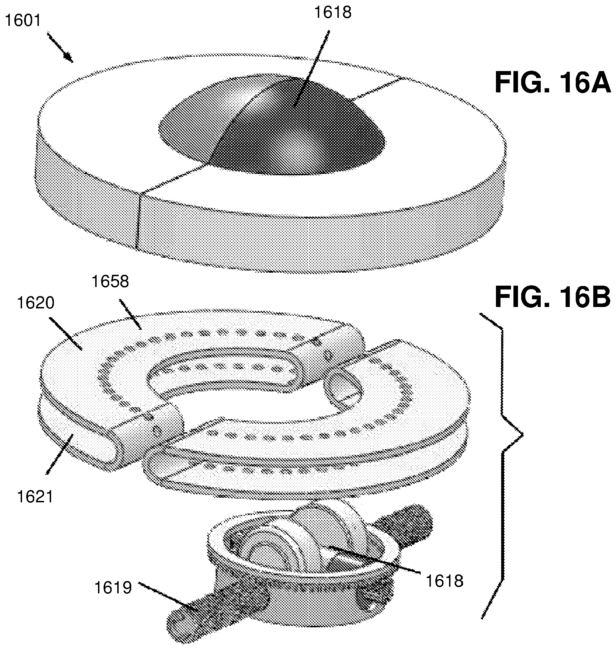

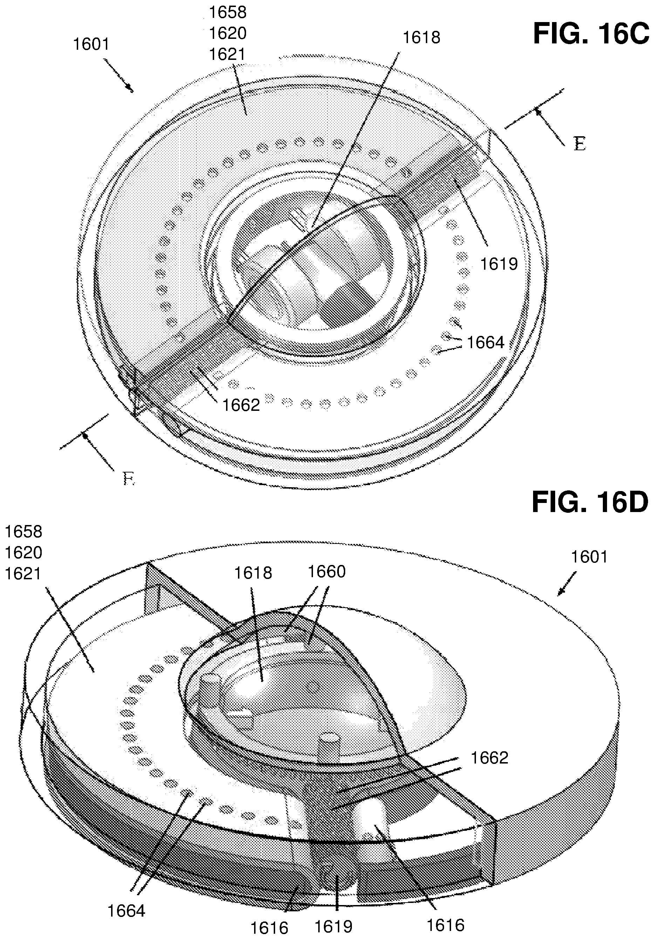

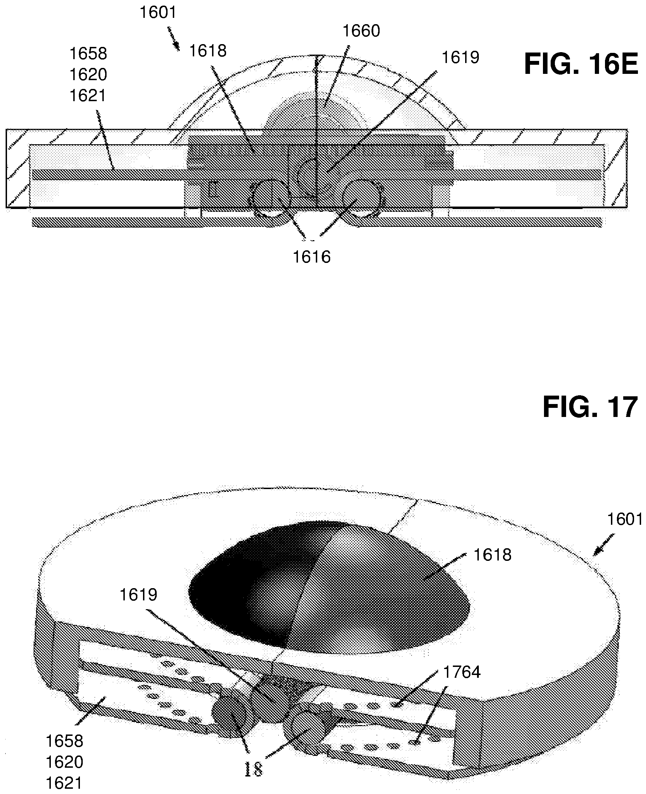

Reference is now made to FIGS. 16A-16E, which illustrate a transdermal delivery device 1601, constructed and operative in accordance with a non-limiting arrangement of the disclosure.

In some arrangements, transdermal delivery device 1601 includes a substrate 1658 is driven by a driver 1518. Optionally, substrate 1658 includes an active surface 1620 including a substance adapted for transdermal delivery. For example, active surface 1620 may include commercially available transdermal patch impregnated or otherwise provided with a substance (e.g., drug). In some arrangements, active surface 1620 includes a patch mounted on a substrate 1658, for example with and adhesive. Alternatively or additionally, active surface 1620 may be an intrinsic part of substrate 1658.

In some arrangements, driver 1618 moves active surface 1620 to one or more of a plurality of operational relationships with the skin of the subject, including active surface 1620 being in fully engaged with the skin (i.e., the entire active area of the active surface 1620 is in contact with the skin), being in partially engaged with the skin (i.e., only a portion of the active surface 1620 is in contact with the skin), and not being in engaged with the skin. In one arrangement, the operation of driver 1618 is controlled by a controller 1660 (FIG. 16D). For example, controller 1660 governs activation, deactivation and/or the speed of driver 1618. Optionally, by controlling driving 1618, controller 1660 controls when and for how long active surface 1620 is in a particular operational relationship with the skin (e.g., at what time of the day is active surface 1620 in full, partial or no contact with the skin and/or for what duration of time).

In some arrangements, transdermal delivery device 1601 may be provided by a manufacturer as a hermetically sealed assembly. Optionally active surface 1620 may be exposed inside the sealed assembly without a linear. Alternatively or additionally, active surface 1620 may have a backing that. For example a backing may protect active surface 1620 from the outer environment. Alternatively or additionally, active surface 1620 may have a liner. For example, the liner may be removed prior to loading substrate 1658 into device 1601. In some arrangements, for example where active surface is includes a discrete patch, a liner may be removed from the patch and the patch may then be mounted onto substrate 1658 and/or the patch may be mounted onto substrate 1658 and then the liner removed. One optional way of removing such a liner is described herein below with reference to FIGS. 21A-21C. Optionally, active surface 1620 may include a permeation enhancer for enhancing delivery of the substance through the stratum corneum.

In some arrangements, driver 1618 and/or controller 1660 may control and/or vary the amount of pressure applied to active surface 1620 against the skin. For example, increasing the pressure between active surface 1620 and the skin may increase the rate of substance delivery per unit time. In some arrangements, driver 1618 and/or controller 1660 may stabilize and/or control the transfer rate of the substance from active surface 1620 by causing the active surface 1620 to contact fresh areas on the skin. In some arrangements, contacting fresh skin may compensate for degradation of the substance concentration and/or absorption in older or previous areas on the skin.

In some arrangements, substrate 1658 is a continuous belt rotatably mounted on a roller 1616. For example, as illustrated in Fig. ID, driver 1618 may be a motor with a transmission (e.g. output shaft) 1619 that meshes with the movable element (e.g., roller) 1616 or directly with substrate 1658. For example, movable element 1616 may include a gear and output shaft 1619 may be complimentary shaped (e.g., having worm threads) to mesh with and turn movable element 1616. Alternatively, output shaft 1619 may be linked to movable element 1616 via a friction drive.

In some arrangements, output shaft 1619 may be linked to movable element 1616 via a male-female connection. For example, output shaft 1619 may be provided with pins or teeth 1662 (male part) that engage holes 1664 (female part) formed in substrate 1658. Teeth 1662 and holes 1664 are also referred to as drive interface elements. Optionally, as driver 1618 turns output shaft 1619, substrate 1658 is caused to turn about rollers 1616.

In some arrangements, substrate 1658 includes an active surface, for example active surface 1620 and a secondary zone 1621. Secondary zone 1621 may be drug free zone in substrate 1658. Alternatively or additionally secondary zone 1621 may include a second drug and/or be configured for a therapeutic process. For example, zone 1621 may include a stratum corneum resurfacing element (for example for "skin activation" or "skin resurfacing"). Optionally, the stratum corneum resurfacing element may include an adhesive surface operative to attach to and peel a portion of stratum corneum. Alternatively or additionally, the stratum corneum resurfacing element may include a chemical (e.g., glycolic acid or salicylic acid) operative to peel a portion of stratum corneum. Alternatively or additionally to any combination of the above, the stratum corneum resurfacing element may include a micro-needle array.

In some arrangements, as substrate 1558 rolls onto the skin, active surface 1620 sometimes fully contacts the skin (at which time secondary zone 1621 may not contact the skin at all, but alternatively can be configured to partially or fully contact the skin, too), sometimes partially contacts the skin (at which time secondary zone 1621 may partially contact the skin, but alternatively can be configured for no contact or full contact) and sometimes does not contact the skin at all (at which time the secondary zone 1621 may fully contact the skin (but alternatively can be configured for no contact or partial contact).

In some arrangements, active surface 1620 and secondary zone 1621 move synchronously with each other. For example, in the illustrated arrangement, active surface 1620 and secondary zone 1621 form are arranged longitudinally on a continuous belt. Optionally two such belts (mirror images of each other) are arranged to roll on two rollers 1616. The substrates 1658 are optionally configured to form two halves of a round, compact device 1601. In some arrangements, the substrates 1658 are located peripherally outwards of driver 1618. For example, one driver 1618 is operative to drive more than one substrate 1658.

In some arrangements, controller 1660 (Fig. ID) may be provided internally in (or externally to) device 1601. Optionally, controller 1660 controls operation of driver 1618. Optional controller 1660 may be preprogrammed to activate driver 1618 continuously, incrementally or a combination thereof in accordance with a treatment plan. In some arrangements, battery (not shown) may be used to energize driver 1618.

In some arrangements, substrate 1658 can be also driven manually by rotating the cover of the device 1601 which may be connected to the drive mechanism (e.g., gear, friction, male-female drive).

In one arrangement, roller 1616 has a perimeter larger than the size (rolling length) of substrate 1658. For example, in this manner, the moving and rolling speeds of the device may be synchronized.

In some arrangements, other methods may be implemented in the invention for moving an active surface with a driver to change the amount of active area engaged with the skin. For example, in some arrangements, a patch is mounted on a substrate but not on a roller. Optionally the driver is operative to grasp and/or move the substrate. For example the substrate may be arranged to be lifted off the skin by the driver, such as being moved perpendicularly off the skin. For instance the driver may lift the substrate away from the skin may be by gradually peeling at an angle and/or lifting the patch off the skin, and then afterwards reattaching the patch to the skin, either at the same place or a different place. The driver may, for example, include pincers or slender jaws and the like for grasping the substrate and patch. The driver can move the patch linearly and/or rotationally.

Reference is now made to *Fig.* 17, which illustrates an optional set of drive interface elements in accordance with an arrangement of the disclosure. For example, elements enhance torque to move substrate 1658. For example, the additional set of drive interface elements may include another set of holes 1764 that receive torque from driver 1618 and/or move substrate 1658 as in a tracked- wheel mechanism.

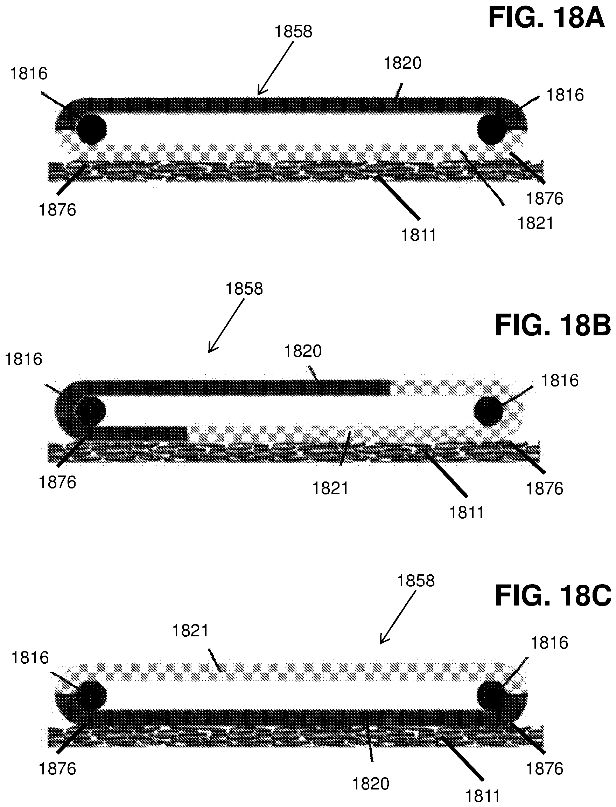

Reference is now made to *FIGS. 18A-18C**,* which illustrate progressive movement of a continuous belt substrate 1858 including an active surface 1820 and a secondary zone 1821 in accordance with an arrangement of the disclosure. Optionally a device may include a single straight continuous track substrate 1858 that rolls along the skin of a subject. Alternatively or additionally, rollers 1816 are
optionally contained by a stationary housing. For example, moving substrate 1858 may engage new portions of the substrate to single area of skin. Optionally active surface 1820 is impregnated with a drug not present in secondary zone 1821. Optionally the portion of the belt positioned away from the skin (e.g. above the roller in the exemplary illustration) is disengaged from the skin. Optionally the portion of the belt positioned against skin (e.g. below the roller in the exemplary illustration) is engaged to the skin. An active surface in the disengaged position is optionally stored for later engagement by further rolling to the engaged position. For example, a disengaged portion (e.g. above the rollers) may be covered by the housing of the device (for example as illustrated by zone 1620 in *FIGs. 16A-16B**.*

In some arrangements moving substrate 1858 may change a rate of drug administration to a subject. For example, in Fig. 18A, active surface 1820 with the substance is above rollers 1816 and secondary zone 1821 is below rollers 1816, as a result active surface 1820 is optionally distanced from and/or fully disengaged from skin 1811 of a subject and/or the drug is not being administered to the subject. In the example of Fig. 3B, substrate 1858 has turned so that active surface 1820 is partially turned below rollers 1816 and secondary zone 1821 is partially above rollers 1816, as a result a portion of active surface 1820 is engaged to and/or in contact with skin 1811 of a subject and/or the drug is administered to the subject at a reduced rate. In Fig. 18C, the substrate 1858 has turned so that active surface 1820 is now below rollers 1816 and secondary zone 1821 is now above rollers 1816, as a result a active surface 1820 is fully engaged to and/or in contact with skin 1811 of a subject and/or the drug is administered to the subject at a full rate. Optionally, a second roller may be supplied whose belt includes a protective liner that contacts substrate 1858 in the stored position. For example, in the case of continuous belt substrate 1858, rollers 1816 may simultaneously peel and/or place substrate 1858 from and to the skin on separation lines 1876.

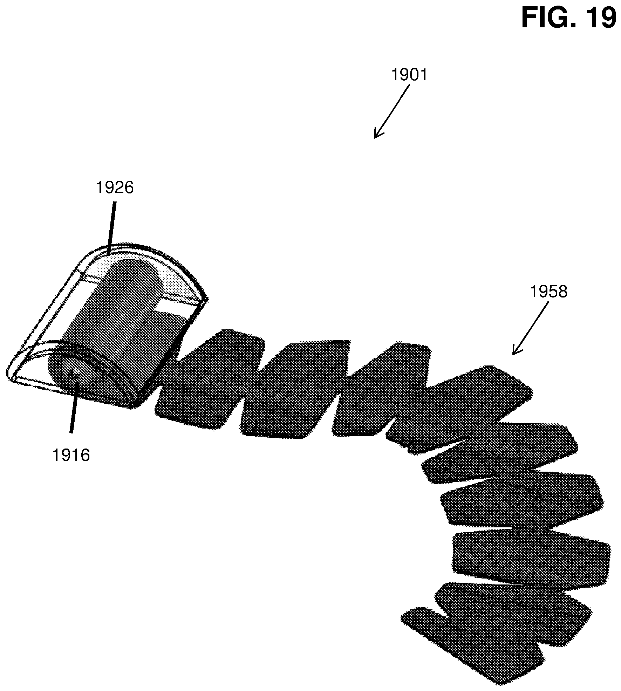

Fig. 19 illustrates a roller patch engagement device 1901 for applying a patch according to an arrangement of the disclosure. In some arrangements, a substrate 1958 is optionally stored in a frame 1926 on a roller 1916. For example, as frame 1926 rolls across the skin of a subject, it engages a further portion of substrate 1958. Optionally, device 1901 is driven by a driver. In some arrangements, substrate 1958 may have active and/or inactive surfaces. For example, the underlying substrate 1958 may be inactive, but medicine patches of one or more kinds may be connected (for example by adhesive) to substrate 1958 in one or more locations. As the substrate is rolled into engagement, the various active surfaces are engaged. Alternatively or additionally, substrate 1958 may be a homogenous patch. For example, device 1901 may constantly roll out new substrate for example to keep a steady dose of the drug over a long time.

In some arrangements, rotation of roller 1916 is driven by a driver. Optionally the driver may drive roller 1916 at a constant rate. For example the dose schedule of the medicine may be determined by the distribution of active surfaces along substrate 1958. Alternatively or additionally, a driver may be controlled by a controller that may be programmed to engage a new section of substrate 1958 at specific times and/or dependent upon events. Alternatively or additionally, device 1901 may be manually operated by pushing along the skin.

In some arrangements, a substrate (for example substrate 1958) may be slotted or otherwise shaped so that device 1901 can engage the substrate along the skin while moving along a curved path and/or a straight path.

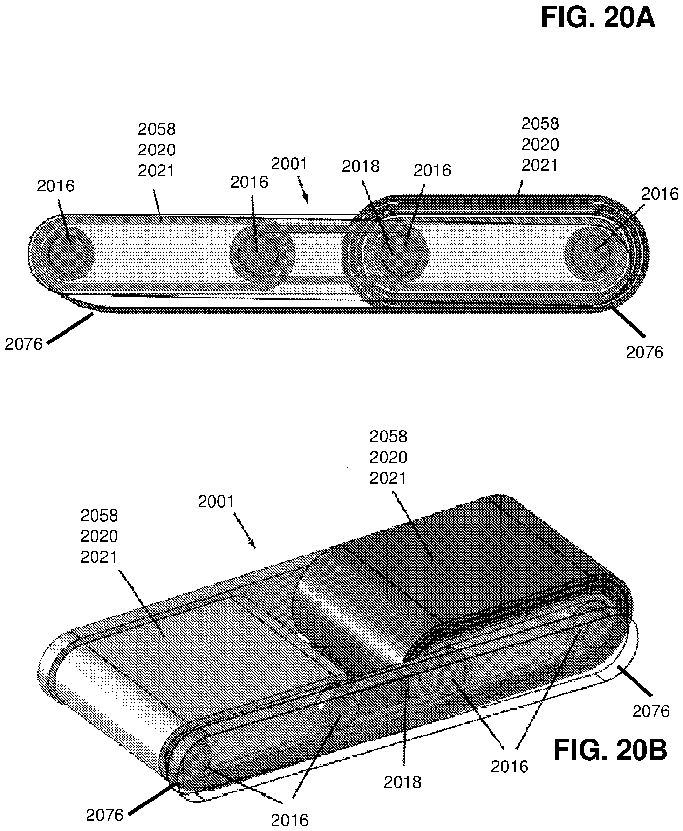

Reference is now made to FIGS. 20A and 20B, which illustrate a transdermal delivery assembly 2001, constructed and operative in accordance with another non-limiting arrangement of the disclosure. In some arrangements, a belt substrate 2058 is wound around two sets of rollers 2016. Substrate 2058 optionally includes one or more zones. For example an active surface 2020 and a secondary zones 2021 (e.g., a stratum corneum resurfacing elements). Optionally, a driver 2018 turns rollers 2016 for driving substrate 2058. One of rollers 2016 is optionally the output shaft of driver 2018. For example, driver 2018 may drive rollers 2016 and/or substrate 2058 directly. For example, rollers 2016 may simultaneously peel and/or place substrate 2058 from and to the skin on separation lines 2076.

Reference is now made to FIGS. 21A-21B, which illustrate a patch engaging device 2101 which peels and lifts a patch to a disengaged position in accordance with an arrangement of the disclosure. In some arrangements substrate 2158 is arranged on rollers 2116a and 2116b. Optionally, rollers 2116a and 2116b are disposed in a housing 2126. For example, rollers 2116a and 2116b may rotate and move linearly
along a track 2146. As rollers 2116a and 2116b move along the skin of a subject in one direction, they optionally disengage the substrate 2158 by peeling substrate 2158 off the skin. As rollers 2116a and 2116b move along the skin of a subject in another direction, they optionally engage the substrate 2158 by placing substrate 2158 onto the skin.

In some arrangements, rollers 2116a and 2116b rotate and/or move along track 2146 to engage and/or disengage substrate 2158 while housing 2126 remains stationary. For example, housing 2126 may be attached to a subject (e.g. with an adhesive). Optionally movement may be driven by a driver and/or controlled by a controller. Alternatively or additionally housing 2126 and rollers 2116a and 2116b may be moved along the subject.

In some arrangements, tension from a puller (for example connection 2180b to frame 2126 and upper roller 2116a is converted by a direction converting element (for example lower roller 2116b and/or for example guide 895 of FIG. 8A) to peel a patch off of skin. For example, guide 895 may include a bumper and/or an arm and/or flat support and/or a rounded support and/or a smooth support.

In some arrangements, a first portion 2121a of substrate 2158 is held stationary (for example at location 2180a on the base of the frame) on and/or near the skin surface of the subject while second portion 2121b is held (for example at location 2180b on frame) distanced from the skin of the recipient. For example, portion 2121a near the skin may be between lto5 mm from the skin and/or between 1 to 0.1 mm of the skin and/or less than 0.1 mm from the skin and/or in contact with the skin. For example, portion 2121b is held stationary distanced from the skin. For example portion 2121b may be between lto5 mm from the skin and/or between 0.1 to 1 mm of the skin and/or more than 5 mm from the skin. For example portion 2121b may be between 10 to 1000 times as far from the skin as portion 2121a and/or between 2 to 10 times as far and/or more than 1000 times as far. Optionally the length of the strip of substrate 2158 between portions 2121a and 2121b is longer than the distance between points 2180a and 2180b. For example the extra length of the strip may leave slack from wrapping the strip in an S shaped curve around rollers 1916.

In some arrangements, substrate 2158 wraps around a pair of rollers 2116a and 2116b in an S-curve. In some arrangements this arrangement saves on space and/or maintains synchronization of the patch and roller motion.

In some arrangements, substrate 2158 wraps around two rollers 2116a and 2116b (for example as illustrated in Fig. 21A-21C). Alternatively or additionally, substrate 2158 wraps around more than two rollers 2116c, 2116d, and 2116e (for example in device 2102 as illustrated in Fig. 21D). Optionally, some of rollers 2116c, 2116d, and 2116e may include a gear 2170. An optional gear train 2171 meshes between gear 2170 and an optional gear rack 2172. In some arrangements, this arrangement provides precise control and synchronization of the patch and roller motion and/or increases torque and/or speed.

In some arrangements, multiple rollers 2116a-2116e facilitates storing substrate 2158 disengaged and spread out distanced from the skin. In some cases this may avoid a potential problem. For example, in some cases where a patch is stored on rolled around a roller, as the patch is laid out on the skin of the subject, the patch unrolls from the roller and sticks to the skin of the subject. As the patch is peeled off the skin, it starts to wrap around the roller. Depending on the diameter of the roller and adhesive properties of the patch (among other factors), if only one roller is used, the patch may stick to itself as it wraps around the roller. This self sticking may make it difficult to unwrap the patch in order to redeploy the patch, engaging it with the skin. An alternative solution may include use of large diameter rollers. Another alternative solution is to place a liner (for example substrate 1558 as illustrated in FIG. 15C and/or protector 922 as illustrated in FIG. 14) on the dorsal face of the patch and/or between layers of the patch as it wraps around the roller. Some arrangements with multiple rollers 2116a-2116e solve this problem by ensuring the patch does not wrap onto itself and/or stick to itself during storage. In some arrangements, multiple rollers may be configured to avoid significantly increasing the size of the device. Optionally, an additional roller or rollers may cause the patch to travel a longer distance when getting peeled off and take the place of a single, large-diameter roller. For example, rollers 2116b and 2116e may peel and/or place substrate 2158 from and/or to the skin on a separation line 2176.

FIGS. 22A-22B illustrate a patch control device that peels the edges of a patch with a shape changing substrate in accordance with an arrangement of the current invention. In some arrangements, bending substrate 2258 has a flattened state which engages a patch 2220 with skin 2211 of a subject for example as illustrated in FIG. 22A.

Substrate 2258 optionally has a bent configuration which lifts an edge of patch 2220 off of skin 2211 in a disengaged state, for example as illustrated in FIG. 22B.

In some arrangements, substrate 2258 includes the surface of a balloon. For example a driver of the device may include a pump which pumps fluid (for example air) through an opening 2270 to inflate the balloon into the disengaged state and/or deflate the balloon into an engaged state. Alternatively or additionally other shape changing substrate for example as nitinol, a joint may be used in place of a balloon. Optionally substrate 2258 is attached to the dorsal face of patch 2220. Alternatively or additionally substrate 2258 may be attached to the edges thereof. For example, substrate 2258 may peel and/or place patch 2220 from and/or to the skin on a curved separation line 2276.

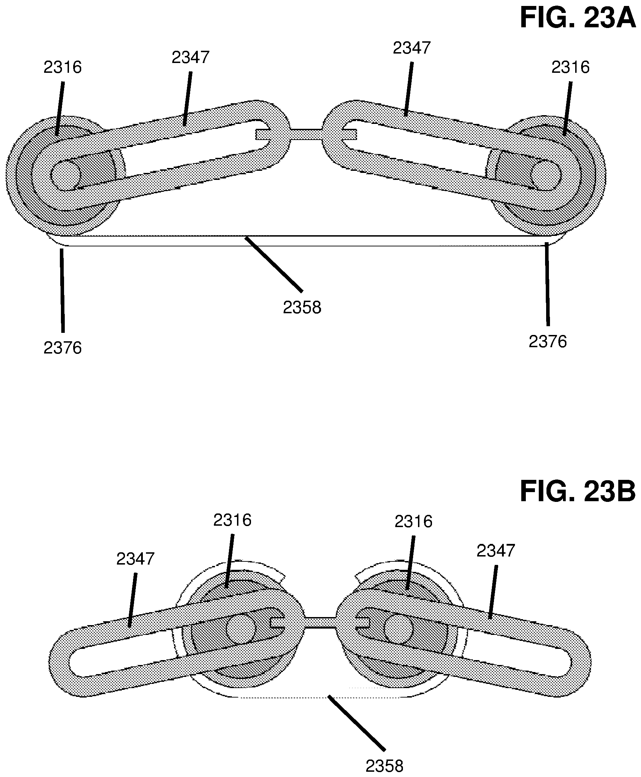

FIGS. 23A-23B illustrate a patch control device with a roller on an angled track that lifts and rolls a patch in accordance with an arrangement of the disclosure. In some arrangements, as a roller 2316 rolls up a substrate 2358, roller 2316 and substrate 2358 are lifted on track 2347 away from skin. In some arrangement multiple rollers 2316 may be mounted on multiple tracks 2347. For example rollers 2316 may act in a synchronized fashion for engaging of substrate 2458 fully from skin (for example as illustrated in FIG. 23A) and/or fully engaging substrate 2358 (for example as illustrated in FIG. 23 B). Alternatively or additionally one roller 2316 may function to partially disengage and/or engage substrate 2358. For example, rollers 2316 may peel and/or place substrate 2358 from the skin on dual separation lines 2376.

FIG. 24 illustrates a ductile substrate accordance with an arrangement of the disclosure. In some arrangements, a substrate 2458 may be hung hangers 2472 and/or supports 2474 from a dorsal side thereof. Optionally an active surface (e.g. a patch) may be located on a ventral face of substrate 2458. Optionally hangers 2472 may be moved dorsally and ventrally to disengage and/or engage the active surface. For example, the movement of hangers 2472 may be in a wave motion to peel the substrate 2458 from the skin along a separation line 2476. In some arrangements placing hangers 2472 and/or moving parts on the dorsal side of a substrate may protect hair of subject from being trapped in the moving parts.

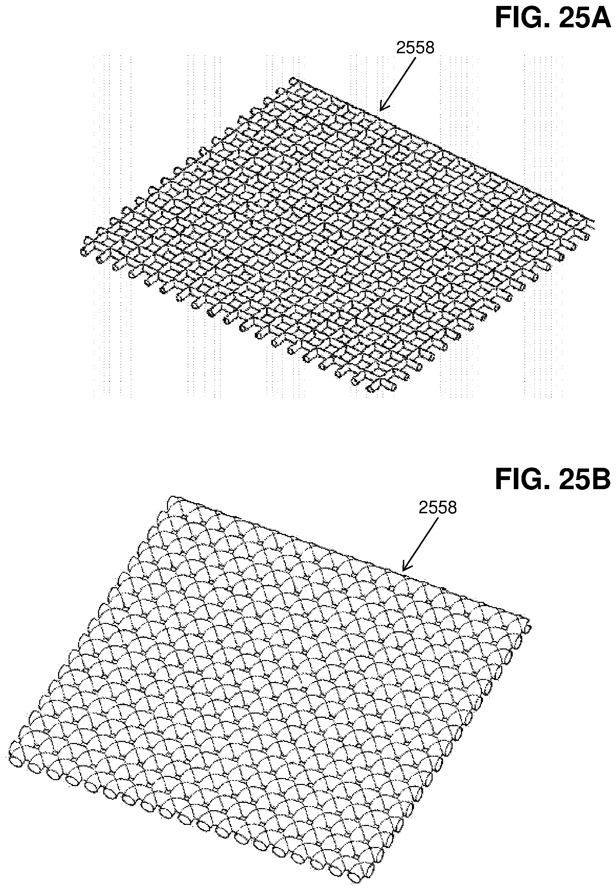

FIGS 25A-25B illustrate an expandable patch control substrate that is placed between a patch and skin. In some arrangements, expanding a substrate 2558 between a patch and skin (for example as illustrated in FIG. 25B) may disengage the patch. In some arrangements, contracting a substrate 2558 between a patch and skin (for example as illustrated in FIG. 25B) may engage the patch. Optionally substrate 2558 may include an inflatable mesh.

As used herein the term "about" refers to ± 5%.

The terms "comprises", "comprising", "includes", "including", "having" and their conjugates mean "including but not limited to".

The term "consisting of means "including and limited to".

The term "consisting essentially of' means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Although the invention has been described in conjunction with specific embodiments thereof, it is evident that many alternatives, modifications and variations will be apparent to those skilled in the art. Accordingly, it is intended to embrace all such alternatives, modifications and variations that fall within the scope of the appended claims.

Citation or identification of any reference in this application shall not be construed as an admission that such reference is available as prior art to the present invention. To the extent that section headings are used, they should not be construed as necessarily limiting.

## Claims

1. A regulator for a transdermal drug administration to a surface of a skin of a subject comprising:
a connector (924) for attachment to a transdermal drug patch (920);
**characterised by** an actuator (915) to mechanically manipulate the transdermal patch (920) with respect to said skin of the subject wherein said manipulating includes at least distancing an active surface of the patch (920) from said skin of the subject and contacting said active surface to said skin;
a housing (926, 1526) comprising a patch control device (1501) that comprises:
a roller (1516) and a frame (1526);
the frame (1526) comprising therein a track (947),
wherein the roller (1516) is arranged to rotate and slide along the track (947) inside the frame (1526) to engage and/or disengage said patch (920); and
a programmable controller operationally connected to said actuator (915) to control said manipulating.

2. The regulator of claim 1, wherein said patch (920) comprises at least one of:
a) a conventional transdermal drug patch (920) configured for administering a drug according to a predetermined dosage schedule;
b) an active surface and a non-active zone and wherein said connector (924) is configured to attach to said patch (920) on said non-active zone; and
c) an area in contact with the skin of the subject and not contacting said connector (924).

3. The regulator of claim 1, further comprising at least one of:
a) an attachment surface configured for attachment to the skin of the subject;
wherein said actuator moves said connector (924) with respect to said attachment surface; and
b) a ventral contact surface configured to attach to a dorsal surface of said patch (920), when said device is placed onto said patch (920).

4. The regulator of claim 1, wherein said housing (926) is configured to adhere to said transdermal drug patch (920) or an adaptor for said transdermal drug patch (920), or to said surface of a skin of a subject or wherein said housing (926) comprises a contact surface configured for contacting a skin of a subject, and wherein said transdermal drug patch (920) or an adaptor for said transdermal drug patch (920) attaches to said contact surface.

5. The regulator of claim 1, wherein said actuator is at least one of:
a) comprising a guide directing a separation line between the patch (920) and the skin;
b) comprising a puller for peeling said patch (920) from said skin,
c) configured to pull said patch (920) at an angle between 70 to 110 degrees relative to said skin's surface; and
d) configured to fully engage, partially engage and disengage said patch (920) from the skin.

6. The regulator of claim 1, further comprising:
a repository for storing a portion of said patch (920) in an unengaged state, wherein storing a portion of said patch (920) in an unengaged state includes rolling the portion around said roller (916).

7. The regulator of claim 1, wherein said roller (916) is configured to move linearly or longitudinally along said track (947).

8. The regulator of claim 6, further comprising at least one of:
a) a non-reactive protector cover for covering an active surface of said patch (920) during storing; and
b) a protector located between layers of said patch (920) wrapped on said roller (916), optionally wherein said protector includes a dorsal surface of said patch (920).

9. The regulator of claim 1, wherein said patch (920) includes an adapter configured to attach to a dorsal face of said patch (920) and said adapter comprises a protector (922).

10. The regulator of claim 1, further comprising:
a synchronizer to synchronize a rate of rotation of the roller (916) with a rate of movement of the roller (916) along the track.

11. The regulator of claim 1, further comprising:
an alignment device for aligning the patch (920) to the device when mounting the patch (920) to the device.

12. The regulator of any one of claims 1 to 11, wherein said controller comprises a computer program product embodied in one or more computer readable mediums having computer readable program code embodied thereon, the program code executable by at least one hardware processor to regulate transdermal drug administration to a subject via a transdermal drug patch (920).

13. The regulator of claim 12, wherein said computer readable program code is further executable to regulate administration to a subject by at least one of:
a) restart said administration at a predetermined time after said interrupting;
b) sense an event and wherein said interrupting or initiating is in response to said event, optionally wherein said event is related to a reaction of said subject to said drug;
c) initiate a new administering of a second drug automatically after said administering;
optionally wherein said initiating a new administering includes exposing a new zone of said patch (920) to a skin of said subject; and
d) increase or decrease said modified rate schedule in response to an event, optionally
wherein said increasing is by increasing an area of contact between an active zone of said patch (920) and a skin of said subject and said decreasing is by decreasing an area of contact between an active zone of said patch (920) and a skin of said subject.

14. The regulator of claim 12, wherein said computer readable program code is further executable to regulate administration to a subject by at least one of:
a) interrupt said administrating automatically before administration of 100% of a dosage of said patch (920);
b) automatically initiate or interrupt said administering of said drug at a predetermined time; and
c) administer said drug to said subject at a modified rate schedule, having a dosage rate less than a dosage rate of said patch (920).

## Patentansprüche

1. Regulator für eine transdermale Arzneimittelverabreichung an der Hautoberfläche eines Subjekts, umfassend:
ein Verbindungselement (924) zur Anbringung an einem transdermalen Arzneimittelpflaster (920),
**gekennzeichnet durch** ein Betätigungselement (915), um das transdermale Pflaster (920) bezüglich der Haut des Subjekts mechanisch zu manipulieren,
wobei das Manipulieren wenigstens das Entfernen einer aktiven Oberfläche des Pflasters (920) von einer Haut des Subjekts und/oder das Kontaktieren der aktiven Oberfläche mit der Haut beinhaltet,
ein Gehäuse (926, 1526), umfassend eine Pflastersteuervorrichtung (1501), die umfasst:
eine Rolle (1516) und einen Rahmen (1526),
wobei der Rahmen (1526) darin eine Führungsschiene (947) umfasst,
wobei die Rolle (1516) dazu beschaffen ist, sich zu drehen und entlang der Führungsschiene (947) innerhalb des Rahmens (1526) zu gleiten, so dass sie mit dem Pflaster (920) in Eingriff kommt und/oder sich davon löst, und
eine programmierbare Steuerung, die wirksam mit dem Betätigungselement (915) verbunden ist, um das Manipulieren zu steuern.

2. Regulator nach Anspruch 1, wobei das Pflaster (920) wenigstens eines umfasst von:
a) ein herkömmliches transdermales Arzneimittelpflaster (920), das zum Verabreichen eines Arzneimittels gemäß einem vorgegebenen Dosierungsplan beschaffen ist,
b) eine aktive Oberfläche und eine nicht-aktive Zone und wobei das Verbindungselement (924) dazu beschaffen ist, an dem Pflaster (920) auf der nicht-aktiven Zone anzuliegen, und
c) eine Fläche, die in Kontakt mit der Haut des Subjekts kommt und nicht das Verbindungselement (924) kontaktiert.

3. Regulator nach Anspruch 1, ferner umfassend wenigstens eines von:
a) eine Anbringungsfläche, die zur Anbringung auf der Haut des Subjekts beschaffen ist,
wobei das Betätigungselement das Verbindungselement (924) in Bezug auf die Anbringungsfläche bewegt, und
b) eine ventrale Kontaktfläche, die dazu beschaffen ist, an einer dorsalen Fläche des Pflasters (920) angebracht zu werden, wenn die Vorrichtung auf dem Pflaster (920) angeordnet wird.

4. Regulator nach Anspruch 1, wobei das Gehäuse (926) dazu beschaffen ist, an dem transdermalen Arzneimittelpflaster (920) oder einem Adapter für das transdermale Arzneimittelpflaster (920) oder an der Hautoberfläche eines Subjekts zu haften oder wobei das Gehäuse (926) eine Kontaktfläche umfasst, die zum Kontaktieren einer Haut eines Subjekts beschaffen ist, und wobei das transdermale Arzneimittelpflaster (920) oder ein Adapter für das transdermale Arzneimittelpflaster (920) an der Kontaktfläche anliegt.

5. Regulator nach Anspruch 1, wobei das Betätigungselement wenigstens eines von:
a) eine Führung umfasst, die eine Trennlinie zwischen dem Pflaster (920) und der Haut lenkt,
b) einen Abzieher zum Abziehen des Pflasters (920) von der Haut umfasst,
c) dazu beschaffen ist, das Pflaster (920) in einem Winkel zwischen 70 bis 110 Grad relativ zu der Hautoberfläche abzuziehen, und
d) dazu beschaffen ist, das Pflaster (920) vollständig mit der Haut in Eingriff zu bringen, teilweise mit der Haut in Eingriff zu bringen und es von der Haut abzulösen.

6. Regulator nach Anspruch 1, ferner umfassend:
ein Behältnis zum Lagern eines Teils des Pflasters (920) in einem freien Zustand, wobei das Lagern eines Teils des Pflasters (920) in einem freien Zustand das Rollen des Teils um die Rolle (916) beinhaltet.

7. Regulator nach Anspruch 1, wobei die Rolle (916) dazu beschaffen ist, sich linear oder längs entlang der Führungsschiene (947) zu bewegen.

8. Regulator nach Anspruch 6, ferner umfassend wenigstens eines von:
a) eine nicht-reaktive Schutzabdeckung für das Abdecken einer aktiven Oberfläche des Pflasters (920) während der Lagerung und
b) eine Schutzvorrichtung, die zwischen Schichten des auf die Rolle gewickelten Pflasters (920) angeordnet ist, wobei die Schutzvorrichtung optional eine dorsale Fläche des Pflasters (920) beinhaltet.

9. Regulator nach Anspruch 1, wobei das Pflaster (920) einen Adapter beinhaltet, der dazu beschaffen ist, an einer dorsalen Fläche des Pflasters (920) anzuliegen und der Adapter eine Schutzvorrichtung (922) umfasst.

10. Regulator nach Anspruch 1, ferner umfassend:
einen Synchronisierer zum Synchronisieren einer Drehgeschwindigkeit der Rolle (916) mit einer Geschwindigkeit der Rolle (916) entlang der Führungsschiene.

11. Regulator nach Anspruch 1, ferner umfassend:
eine Ausrichtungsvorrichtung zum Ausrichten des Pflasters (920) an der Vorrichtung, wenn das Pflaster (920) an der Vorrichtung angebracht wird.

12. Regulator nach einem der Ansprüche 1 bis 11, wobei die Steuerung ein Computerprogrammprodukt umfasst, das in einem oder mehreren computerlesbaren Medien verkörpert ist, die darin verkörperten computerlesbaren Programmcode aufweisen, wobei der Programmcode durch wenigstens einen Hardwareprozessor ausführbar ist, um die transdermale Arzneimittelverabreichung an ein Subjekt über ein transdermales Arzneimittelpflaster (920) zu regulieren.

13. Regulator nach Anspruch 12, wobei der computerlesbare Programmcode ferner ausführbar ist, um die Verabreichung an ein Subjekt zu regulieren durch wenigstens eines von:
a) Neubeginn der Verabreichung zu einer vorgegebenen Zeit nach dem Unterbrechen,
b) Erfassen eines Ereignisses und wobei das Unterbrechen oder Initiieren als Reaktion auf das Ereignis erfolgt, wobei optional das Ereignis mit einer Reaktion des Subjekts auf das Arzneimittel in Verbindung steht,
c) Initiieren einer neuen Verabreichung eines zweiten Arzneimittels automatisch nach der Verabreichung, wobei optional das Initiieren einer neuen Verabreichung das Aussetzen einer neuen Zone des Pflasters (920) einer Haut des Subjekts beinhaltet, und
d) Erhöhen oder Verringern des modifizierten Ratenplans als Reaktion auf ein Ereignis, wobei optional das Erhöhen des modifizierten Ratenplans durch Vergrößern einer Kontaktfläche zwischen einer aktiven Zone des Pflasters (920) und einer Haut des Subjekts erfolgt und das Verringern durch Verringern einer Kontaktfläche zwischen einer aktiven Zone des Pflasters (920) und einer Haut des Subjekts erfolgt.

14. Regulator nach Anspruch 12, wobei der computerlesbare Programmcode ferner ausführbar ist, um die Verabreichung an ein Subjekt zu regulieren durch wenigstens eines von:
a) Unterbrechen der Verabreichung automatisch vor der Verabreichung von 100 % einer Dosis des Pflasters (920),
b) automatisches Initiieren oder Unterbrechen der Verabreichung des Arzneimittels zu einer vorgegebenen Zeit und
c) Verabreichen des Arzneimittels an das Subjekt mit einem modifizierten Ratenplan, der eine Dosierungsrate aufweist, die geringer ist als eine Dosierungsrate des Pflasters (920).

## Revendications

1. Régulateur pour l'administration d'un médicament transdermique sur une surface de la peau d'un sujet, comprenant :
un connecteur (924) destiné à être fixé à un timbre transdermique de médicament (920) ;
**caractérisé par**
un actionneur (915) pour manipuler mécaniquement le timbre transdermique (920) par rapport à la peau du sujet ;
la manipulation consistant au moins à éloigner une surface active du timbre (920) de la peau du sujet et mettre en contact avec la surface active avec la peau ;
un boîtier (926, 1526) comprenant un dispositif de commande de timbre (1501) ayant :
un rouleau (1516) et un cadre (1526) ;
le cadre (1526) comprenant à l'intérieur une piste (947), le rouleau (1516) étant agencé pour tourner et glisser le long de la piste (947) à l'intérieur du cadre (1526) pour appliquer et/ou enlever le timbre (920) ; et
un contrôleur programmable coopérant avec l'actionneur (915) pour commander la manipulation.

2. Régulateur selon la revendication 1,
dans lequel le timbre (920) est au moins l'un des éléments suivants :
a) un timbre transdermique conventionnel de médicament (920) configuré pour administrer un médicament selon un programme de dosage prédéterminé ;
b) une surface active et une zone non active, le connecteur (924) étant configuré pour se fixer au timbre (920) sur sa zone non active ; et
c) une zone en contact avec la peau du sujet et n'entrant pas en contact avec le connecteur (924).

3. Régulateur selon la revendication 1,
comprenant en outre au moins l'un des moyens suivants :
a) une surface de fixation configurée pour se fixer à la peau du sujet ;
l'actionneur déplaçant le connecteur (924) par rapport à la surface de fixation ; et
b) une surface de contact ventrale configurée pour se fixer à la surface dorsale du timbre (920), lorsque le dispositif est placé sur le timbre (920).

4. Régulateur selon la revendication 1,
dans lequel
le boîtier (926) est configuré pour adhérer au timbre de médicament transdermique (920) ou à un adaptateur du timbre de médicament transdermique (920), ou à cette surface de peau d'un sujet, ou
le boîtier (926) comprend une surface de contact configurée pour entrer en contact avec la peau d'un sujet, et
le timbre de médicament transdermique (920) ou un adaptateur pour le timbre de médicament transdermique (920) se fixe à la surface de contact.

5. Régulateur selon la revendication 1,
dans lequel
l'actionneur est au moins l'un des moyens suivants :
a) un guide dirigeant une ligne de séparation entre le timbre (920) et la peau ;
b) un dispositif de traction pour décoller le timbre (920) de la peau ;
c) ou est configuré pour tirer le timbre (920) selon un angle compris entre 70 et 110 degrés par rapport à la surface de la peau ; et
d) appliquer complètement, appliquer partiellement et enlever le timbre (920) de la peau.

6. Régulateur selon la revendication 1,
comprenant en outre :
un dépôt pour recevoir une partie du timbre (920) dans un état non engagé,
dans lequel
le stockage d'une partie de la pièce (920) dans un état non appliqué consiste à enrouler cette partie autour du rouleau (916).

7. Régulateur selon la revendication 1,
dans lequel
le rouleau (916) est configuré pour se déplacer linéairement ou longitudinalement le long de la piste (947).

8. Régulateur selon la revendication 6,
comprenant en outre au moins l'un des moyens suivants :
a) un couvercle protecteur non réactif pour couvrir une surface active du timbre (920) pendant le stockage ; et
b) une protection placée entre les couches du timbre (920) enroulé sur le rouleau (916), la protection comprenant éventuellement la surface dorsale du timbre (920).

9. Régulateur selon la revendication 1,
dans lequel
le timbre (920) comprend un adaptateur pour se fixer à la face dorsale du timbre (920) et l'adaptateur comprend une protection (922).

10. Régulateur selon la revendication 1,
comprenant en outre :
a) un synchroniseur pour synchroniser la vitesse de rotation du rouleau (916) sur la vitesse de déplacement du rouleau (916) le long de la piste.

11. Régulateur selon la revendication 1,
comprenant en outre :
un dispositif d'alignement pour aligner le timbre (920) sur le dispositif lors de la mise en place du timbre (920) sur le dispositif.

12. Régulateur selon l'une quelconque des revendications 1 à 11,
dans lequel
le contrôleur comprend un produit-programme informatique sur un ou plusieurs supports lisibles par ordinateur ayant un code de programme lisible par ordinateur, le code de programme pouvant être exécuté par au moins un processeur matériel pour réguler l'administration transdermique de médicament à un sujet par l'intermédiaire d'un timbre transdermique de médicament (920).

13. Régulateur selon la revendication 12,
dans lequel
le code de programme lisible par ordinateur est en outre exécutable pour réguler l'administration à un sujet par au moins l'un des moyens suivants :
a) redémarrer l'administration à un moment prédéterminé après son interruption ;
b) détecter un événement auquel l'interruption ou le redémarrage est en réponse, éventuellement l'événement est lié à une réaction du sujet au médicament ;
c) initier une nouvelle administration d'un second médicament automatiquement après l'administration ; facultativement, l'initiation d'une nouvelle administration consiste à exposer une nouvelle zone du timbre (920) à la peau du sujet ; et
d) augmenter ou diminuer le programme de taux modifié en réponse à un événement, éventuellement, l'augmentation se faisant en augmentant la surface de contact entre une zone active du timbre (920) et la peau du sujet et la diminution se faisant en diminuant la surface de contact entre la zone active du timbre (920) et la peau du sujet.

14. Régulateur selon la revendication 12,
dans lequel
le code de programme lisible par ordinateur est en outre exécutable pour réguler l'administration à un sujet par au moins une des actions suivantes :
a) interrompre automatiquement l'administration avant l'administration de 100% d'une dose au timbre (920) ;
b) lancer ou interrompre automatiquement l'administration du médicament à un moment prédéterminé ; et
c) administrer le médicament au sujet selon un programme de taux modifié, avec un taux de dosage plus faible que le taux de dosage du timbre (920).
